# EUROPEAN PATENT SPECIFICATION

(11) **EP 4 499 192 B1**
(45) Date of publication and mention of the grant of the patent: **02.12.2026**
(21) Application number: 23717293.7
(22) Date of filing: 17.03.2023
(51) Int. Cl.: A61M 25/01, A61M 25/06

(54) **STEERABLE INTRODUCER WITH SLIDE BLOCK DIVIDER**
LENKBARE EINFÜHRVORRICHTUNG MIT GLEITBLOCKTEILER
INTRODUCTEUR ORIENTABLE DOTÉ D'UN DIVISEUR DE BLOC COULISSANT

(30) Priority: 25.03.2022 US 202263323744 P; 09.08.2022 US 202263396297 P
(43) Date of publication of application: 05.02.2025
(73) Proprietor: St. Jude Medical, Cardiology Division, Inc., St. Paul, MN 55117 (US)
(72) Inventor: WELTER, Wade, Saint Paul, Minnesota 55117 (US); OLIVERIUS, Andrew, Saint Paul, Minnesota 55117 (US); TEGG, Troy, Saint Paul, Minnesota 55117 (US); HAWKINSON, Neil, Saint Paul, Minnesota 55117 (US); CAJAMARCA, Tobias, Saint Paul, Minnesota 55117 (US)
(74) Representative: Mathys & Squire
(86) International application number: PCT/US2023/015565
(87) International publication number: WO 2023/183202

(56) References cited:
- WO-A1-2017/155867
- WO-A1-2021/120853
- WO-A1-2021/183397
- CN-A- 114 146 289
- CN-A- 114 177 483
- CN-U- 212 940 976
- JP-B2- 6 693 861
- US-A1- 2019 038 873
- US-A1- 2019 111 238
- US-B2- 10 183 149

## Description

### CROSS REFERENCE TO RELATED APPLICATION DATA

The present application claims the benefit under 35 USC §119(e) of U.S. Provisional Application Nos. 63/323,744 filed March 25, 2022 and 63/396,297 filed August 9, 2022.

### FIELD OF DISCLOSURE

This disclosure relates generally to introducers and methods of using introducers. More particularly, the present disclosure relates to handle assemblies for steerable introducers and steerable sheaths methods of manufacturing handle assemblies for steerable introducers and steerable sheaths, and methods of articulating steerable introducers and steerable sheaths.

### BACKGROUND

Catheters are often used for medical procedures such as, for example, diagnostic, therapeutic, and ablative procedures. Typically, a physician advances a catheter through a patient's vasculature to an intended site, such as a site within the patient's heart. A catheter may include one or more electrodes, one or more diagnostic devices, and/or one or more therapeutic devices, which may be used for any suitable medical purpose including, but not limited to, ablation, diagnosis, cardiac mapping, or the like.

For example, ablation therapy may be used to treat various medical conditions. One medical condition in which ablation therapy may be used is the treatment of cardiac arrhythmias. When cardiac tissue is ablated, or at least subjected to ablative energy generated by an ablation generator and delivered by an ablation catheter, lesions form in the cardiac tissue. Electrodes mounted on or in ablation catheters can be used to create tissue necrosis in cardiac tissue to correct conditions such as atrial arrhythmia (including, but not limited to, ectopic atrial tachycardia, atrial fibrillation, and atrial flutter). Arrhythmias can create a variety of dangerous conditions including loss of synchronous atrioventricular contractions and stasis of blood flow. It is believed that the primary cause of atrial arrhythmia is stray electrical signals within the left or right atrium of the heart. An ablation catheter can be used to impart ablative energy (e.g., radiofrequency energy, cryoablation, lasers, chemicals, high-intensity focused ultrasound, etc.) to cardiac tissue to create a lesion in the cardiac tissue that disrupts undesirable electrical pathways and thereby limits or prevents stray electrical signals that lead to arrhythmias.

As another example, electroporation is a non-thermal ablation technique in which electric-fields are applied to induce pore formation in cellular membranes. The electric field may be applied in a pulse train of relatively short duration pulses that last, for example, from a nanosecond to several milliseconds. When electroporation is applied to tissue in an in vivo setting, the cells in the tissue are subjected to a trans-membrane potential to induce the pore formation in the cellular membranes. Electroporation may be reversible (i.e., the temporally-opened pores will reseal) or irreversible (i.e., the pores will remain open), causing cellular destruction. For example, in the field of gene therapy, reversible electroporation is used to accommodate transportation of high molecular weight therapeutic vectors into cells. In other therapeutic applications, irreversible electroporation can be used to induce cell destruction.

Catheters may be introduced through an introducer or sheath. An introducer can be advanced through a patient's vasculature and a diagnostic and/or treatment catheter is then advanced through a lumen of the introducer to the intended site within the patient. The introducer itself may be advanced over a guide wire. The introducer can have an outer diameter small enough to be advanced through the patient's vasculature and a lumen large enough to accommodate advancement of a catheter through the lumen.

A catheter, such as a diagnostic catheter, a treatment catheter, or an introducer can include a steerable section that can be selectively articulated to enhance advancement of the catheter along a path (which is often tortuous) though the patient's vasculature. Typically, the steerable section is located near the distal end of the catheter and one or more pull cables are employed to transmit steering forces from a proximal handle assembly to the steerable section. Examples of introducer with steerable sections are disclosed in U.S. Patent Nos. 4,817,613 and 7,914,515. U.S. Patent Application No. 2019/0111238 A1 discloses a reusable catheter handle system.

### BRIEF SUMMARY

The aforementioned objectives are achieved according to the invention by means of a steerable introducer according to claim 1. Preferred embodiments are defined in the dependent claims.

A steerable introducer including a handle assembly with slide members and a slide block divider for constraining slide members is described herein. The steerable catheter includes an elongated body including a proximal end, a steerable section, a first deflection lumen extending between the proximal end and the steerable section, and a second deflection lumen extending between the proximal end and the steerable section. Within the first deflection lumen, a first deflection wire is disposed and actuatable to induce deflection of the steerable section of the elongated body in a first direction. Within the second deflection lumen, a second deflection wire is disposed and actuatable to induce deflection of the steerable section of the elongated body in a second direction.

A handle assembly can be coupled to the proximal end of the elongated body. The handle assembly includes a handle housing, a slide chassis disposed within the handle housing, a first slide member drivingly coupled with the first deflection wire, a second slide member drivingly coupled with the second deflection wire, and a slide block divider disposed within the handle housing. An actuation assembly can be drivingly coupled with each of the first slide member and the second slide member. The actuation assembly is operable to simultaneously drive the first slide member distally and the second slide member proximally within the slide compartment, or vice-versa.

The slide block divider and the slide chassis combine to form a slide compartment in which each of the first slide member and the second slide member is slidably disposed. Advantageously, the slide block divider can prevent the first deflection wire and the second deflection wire from crossing each other within the handle housing. The slide block divider can constrain each of the first slide member and the second slide member during translation within the slide compartment to inhibit motion transverse to a longitudinal direction of the slide compartment. The slide block divider is shaped and sized to cooperate with an inner surface of the handle housing facilitating a compact housing assembly. Also, as the slide block divider prevents the deflection wires from cross each other and slides from rising within the housing, no extra force may be applied to the actuation assembly to overcome torque or resistance from deflection wires or the slide members. As such, the user can control the deflection of the elongated shaft section using a single hand. As such, the slide block divider can provide an improved handling of the steerable introducer during medical procedures such as, for example, diagnostic, therapeutic, and ablative procedures.

The slide chassis includes a slide member opening extending along a length of the slide chassis. The opening is configured to accommodate installation of at least one of the first slide member and the second slide member into the slide chassis. The slide chassis includes support edges configured to support the slide block divider. The slide member opening is at least partially defined by the support edges. The slide chassis includes shoulders for locating the slide block divider. The shoulders extend in a plane perpendicular to the support edges at an end close to the distal end of the slide chassis. The slide chassis is substantially cylindrical in shape.

The slide block divider includes a fin extending toward the slide members to prevent the slide members from rotating and to prevent the first deflection wire from crossing with the second deflection wire within the handle housing. The fin is a radially projecting portion that extends from a proximal end of the slide block divider to a distal end of the slide block divider and is disposed between the first slide member and the second slide member. The slide block divider includes an external surface interfaced with an inner surface of the handle housing. The inner surface of the handle housing has a curved shape with one or more ribs spaced from each other and extending along the curved shape. Accordingly, the external surface of the slide block divider has a corresponding curved shape with slots to receive the one or more ribs of the inner surface of the handle housing and to prevent relative motion of the slide block divider with respect to the handle.

The slide block divider can include a pair of rails. Each of the pair of rails extends along a length of the slide block divider and is configured to rest on the slide chassis. The slide block divider increases side walls extending along a length of the slide block divider. Each of the pair of rails is formed on a respective one of the side walls. The side walls of the slide block divider comprise a tail portion configured to butt against a shoulder of the slide chassis to facilitate assembly and to prevent relative motion between the slide block divider and the slide chassis. The slide chassis includes flat side portions extending at least partially along a length of the slide chassis, and inner surfaces of the side walls of the slide block divider contact the flat side portions.

The slide block divider has an extended portion at a proximal end of the slide block divider. The extended portion is received in the gap and prevents the first deflection wire from crossing into the second deflection wire during operation of the steerable introducer. The slide chassis includes a slide member opening extending along a length of the slide chassis. The slide member opening is configured to accommodate installation of at least one of the first slide member and the second slide member into the slide chassis.

The first slide member has an external right-handed thread, and the second slide member has an external left-handed thread. The actuation assembly comprises a rotatable knob having internal threads configured to engage the external right-handed thread and the external left-handed thread. The first slide member includes first slide member distal end side surfaces configured to slide along first slide member side surfaces of the slide block divider to inhibit rotation of the first slide member within the slide compartment. The second slide member includes second slide member distal end side surfaces configured to slide along second slide member side surfaces of the slide block divider to inhibit rotation of the second slide member within the slide compartment. The first slide member includes a first deflection wire channel through which the first deflection wire extends, and the second slide member includes a second deflection wire channel through which the second deflection wire extends. The first slide member and the second slide member are separated by a gap in which a portion of the slide block divider extends.

In some embodiments, the handle assembly further includes a first pin assembly having a first pin disposed at a proximal end of the first deflection wire channel of the first slide member and a second pin assembly having a second pin disposed at a proximal end of the second deflection wire channel of the second slide member. The first deflection wire is coupled to the first pin and the second deflection wire is coupled to the second pin. The handle assembly further includes a slide block divider having a fin extending radially toward the slide members. The fin has a height extending radially down to and above the first pin and the second pin. The height of the fin is constant along a longitudinal axis of the slide block divider. In some embodiments, the fin has a tapered shape to prevent the first pin and the second pin from coming in contact with the fin during sliding of the slide members. In some embodiments, each of the first pin and the second pin includes a textured circumferential surface.

The forgoing general description of the illustrative implementations and the following detailed description thereof are merely exemplary aspects of the teachings of this disclosure, and are not restrictive.

### BRIEF DESCRIPTION OF THE DRAWINGS

The accompanying drawings, which are incorporated in and constitute a part of the specification, illustrate one or more embodiments and, together with the description, explain these embodiments. The accompanying drawings have not necessarily been drawn to scale. Any values dimensions illustrated in the accompanying graphs and figures are for illustration purposes only and can or cannot represent actual or preferred values or dimensions. Where applicable, some or all features cannot be illustrated to assist in the description of underlying features. In the drawings:
FIG. 1 illustrates an introducer that includes a steerable section and a handle assembly operable to articulate the steerable section, according to some embodiments of the present disclosure.
FIG. 2A is a close-up view illustration of the handle assembly of FIG. 1.
FIG. 2B is an exploded view illustration of the handle assembly of FIG. 1.
FIG. 3 illustrates a housing portion of the handle assembly of FIG. 1.
FIG. 4A and FIG. 4B illustrate a slide chassis of the handle assembly of FIG. 1.
FIG. 5A and FIG. 5B illustrate slide members of the handle assembly and pull cables of the introducer of FIG. 1.
FIG. 6A, FIG. 6B, FIG. 6C, FIG. 6D, FIG. 6E, and FIG. 6F illustrate a slide block divider of the handle assembly of FIG. 1.
FIG. 7A illustrates the handle assembly of FIG. 1 without the slide block divider over slide members.
FIG. 7B illustrates the handle assembly of FIG. 1 with the slide block divider in place over the slide members.
FIG. 7C is a front cross-sectional view of the handle assembly of FIG. 1 illustrating a slide compartment between slide chassis and the slide block divider.
FIG. 7D is a front cross-sectional view of the handle assembly of FIG. 1 showing the slide members assembled in the slide compartment of FIG. 7C.
FIG. 8 is a cross-sectional view of the handle assembly of FIG. 1 along a longitudinal axis of the handle assembly.
FIG. 9 is a partial view illustration of a handle assembly including a slotted slide and a pin.
FIG. 10A illustrates another example of a slide chassis of the handle assembly of an introducer that includes a steerable section, according to some embodiments of the present disclosure.
FIG. 10B and FIG. 10C illustrate slide members configured to be assembled with the slide chassis of FIG. 10A.
FIG. 10D illustrates a pin that is coupled to each of the slide members of FIG. 10B and FIG. 10C in a handle assembly of a introducer that includes a steerable section.
FIG. 11 illustrates another slide block divider of a handle assembly of an introducer that includes a steerable section, according to some embodiments of the present disclosure.
FIG. 12 is a partial view illustration of a handle assembly that includes the slide block divider of FIG. 11.
FIG. 13 is a diagrammatic illustration of the use of a catheter passed through an introducer that includes a steerable section and a handle assembly operable to articulate the steerable section in a procedure on a patient, according to some embodiments of the present disclosure.
FIG. 14 is a schematic and block diagram view of a system for electroporation therapy, according to some embodiments of the present disclosure.
FIG. 15 illustrates a pin assembly coupled to slide members of the handle assembly of the introducer of FIG. 1.
FIG. 16 is a perspective view of the pin assembly of FIG. 15.
FIG. 17 is a bottom perspective view of another example of a slide block divider, according to some embodiments of the present disclosure.
FIG. 18 is a front view of the slide block divider of FIG. 17.
FIG. 19 is a front cross-sectional view of the handle assembly of FIG. 1 showing the slide block divider of FIG. 17.
FIG. 20 is a cross-sectional view of the handle assembly of FIG. 1 with the slide block divider of FIG. 17.
FIG. 21 and FIG. 22 are examples of a pin with textured circumferential surface usable in the pin assembly of FIG. 16.

### DETAILED DESCRIPTION

The description set forth below in connection with the appended drawings is intended as a description of various embodiments of the disclosed subject matter and is not necessarily intended to represent the only embodiment(s). In certain instances, the description includes specific details for the purpose of providing an understanding of the disclosed embodiment(s). However, it will be apparent to those skilled in the art that the disclosed embodiment(s) can be practiced without those specific details. In some instances, well-known structures and components can be shown in block diagram form in order to avoid obscuring the concepts of the disclosed subject matter.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics can be combined in any suitable manner in one or more embodiments. Further, it is intended that embodiments of the disclosed subject matter cover modifications and variations thereof.

It is to be understood that terms such as "distal," "proximal," "top," "bottom," "front," "side," "length," "inner," and the like that can be used herein merely describe points of reference and do not necessarily limit embodiments of the present disclosure to any particular orientation or configuration. As used herein, "proximal" refers to a direction toward the end of a catheter or an introducer near the clinician and "distal" refers to a direction away from the clinician and (generally) inside the body of a patient. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation.

The terms "longitudinal," "axial" or "axially" are generally longitudinal as used herein to describe the relative position related to an introducer, a catheter, a introducer handle, or other components of the system herein. For example, "longitudinal" or "axial" indicates an axis passing along a center of a introducer or a catheter from a proximal end to a distal end, or along a center of the introducer handle from a proximal end to a distal end. The term "radial" generally refers to a direction perpendicular to the "axial" direction.

Introducers that include a steerable section and a handle assembly operable for articulating the steerable section are described herein. The catheters can be used in any suitable medical procedure including, but not limited to, diagnostic and/or therapeutic procedures. While the handle assemblies may be described herein as included in an introducer with a steerable section, the handle assemblies can be included in any suitable type catheter such as, for example, diagnostic catheters, therapeutic catheters, and introducer.

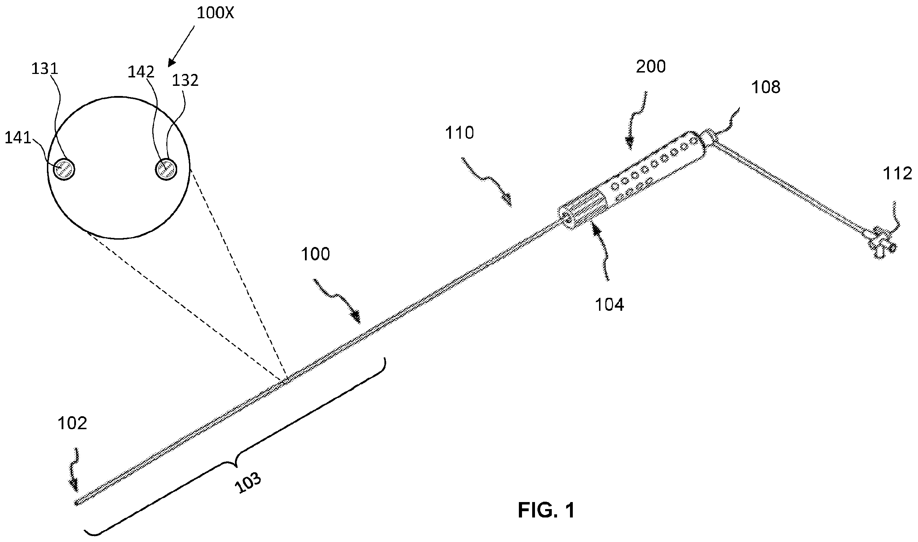

Turning now to the figures in which similar reference identifiers are associated with similar elements, FIG. 1 shows an introducer 100 according to some embodiments of the present disclosure. The introducer 100 includes an elongated introducer shaft 110 having a distal end 102, a steerable section 103 disposed adjacent to the distal end 102, and a proximal end 104. The introducer 100 can be configured as any suitable type of catheter including, for example, an introducer, a diagnostic catheter, or a treatment catheter. The introducer 100 includes a handle assembly 200, which is coupled with the introducer shaft 110 at the proximal end 104. As described herein, the handle assembly 200 is operable to selectively articulate the steerable section 103 to accommodate advancement of the introducer shaft 110 through a patient's vasculature and/or retraction of the introducer shaft 110 from the patient's vasculature. In the illustrated embodiment, the introducer 100 is configured as an introducer and further includes a connection hub 108 operably connected to an inner lumen (not shown) within the handle assembly 200 for insertion of another catheter (e.g., a diagnostic catheter, a treatment catheter), fluids, or any other suitable medical substances or medical devices known to those of ordinary skill in the art. The introducer 100 further includes a valve 112 operably connected to the connection hub 108. An example of an introducer is discussed in detail in U.S. patent 7,914,515.

Referring now to cross-section portion 100X shown in FIG. 1, the introducer shaft 110 includes a first deflection lumen 131, a first deflection wire 141, a second deflection lumen 132, and a second deflection wire 142. Each of the first deflection lumen 131 and the second deflection extends between the proximal end 104 and the steerable section 103 (or the distal end 102). The first deflection wire 141 extends within the first deflection lumen 131. The first deflection wire 141 is actuatable via the handle assembly 200 to induce deflection of the steerable section 103 in a first direction (e.g., left or up). The second deflection wire 142 extends within the second deflection lumen 132. The second deflection wire 142 is actuatable via the handle assembly 200 to induce deflection of the steerable section 103 in a second direction (e.g., right or down), opposite of the first direction.

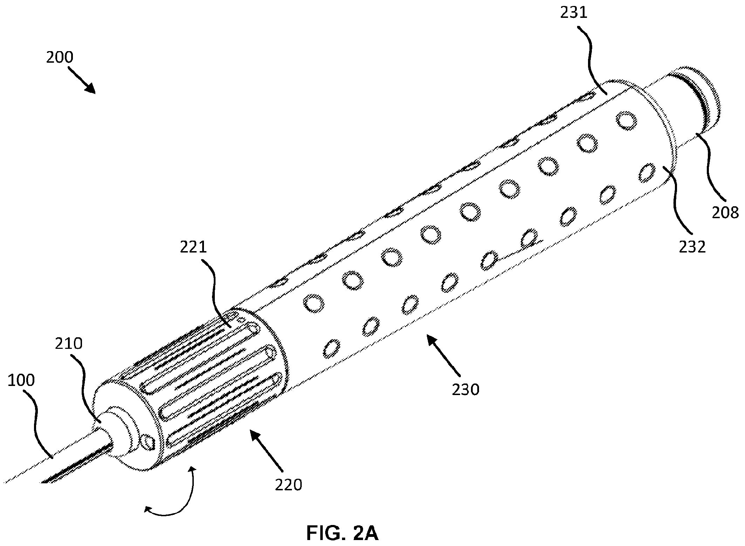

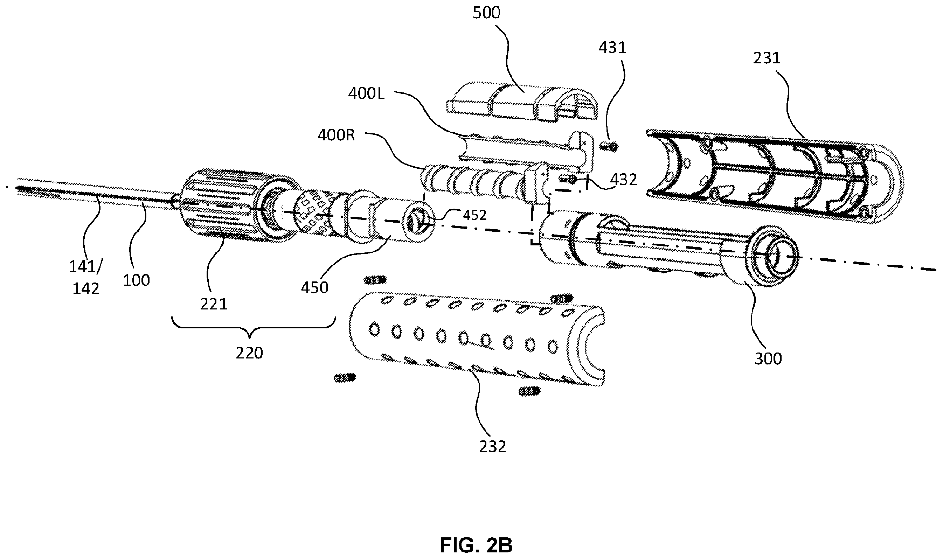

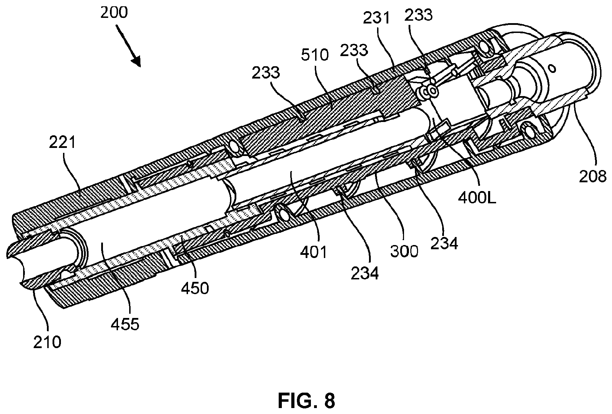

FIG. 2A shows a close-up view of the handle assembly 200. FIG. 2B shows an exploded view of the handle assembly 200. FIG. 8 shows a longitudinal cross-sectional view of the handle assembly 200 showing example assembly of components therein.

Referring to FIG. 2A, the handle assembly 200 includes a handle housing 230 and an actuation assembly 220 axially coupled to a handle housing 230. The actuation assembly 220 includes a rotatable knob 221. In some embodiments, the handle housing 230 includes a first housing portion 231 and a second housing portion 232 that is removably coupled to the first housing portion 231. The handle assembly 200 is coupled to the proximal end 104 of the introducer shaft 110. The handle assembly 200 includes a retaining nut 210 used to secure the introducer shaft 110 to the handle assembly 200. The introducer shaft 110 extends through the retaining nut 210 to an actuation hub 450 (shown in FIG. 2B). The handle assembly 200 can include a connector 208 at the proximal end of the handle assembly 200 instead of the connection hub 108. For example, in some embodiments the connector 208 is an electrical connector configured to be operatively coupled with a mating electrical connector coupled with a recording or monitoring device. The handle assembly 200 may include a channel 401/411 (see FIG. 8) through which an electrical wire tube (e.g., containing electrical wires exiting the introducer shaft 110) may extend to the connector 208.

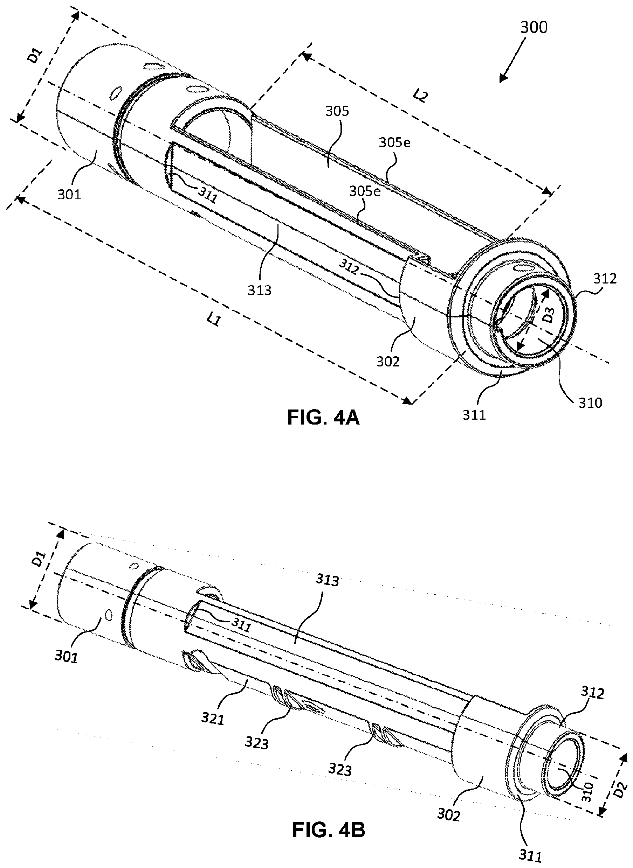

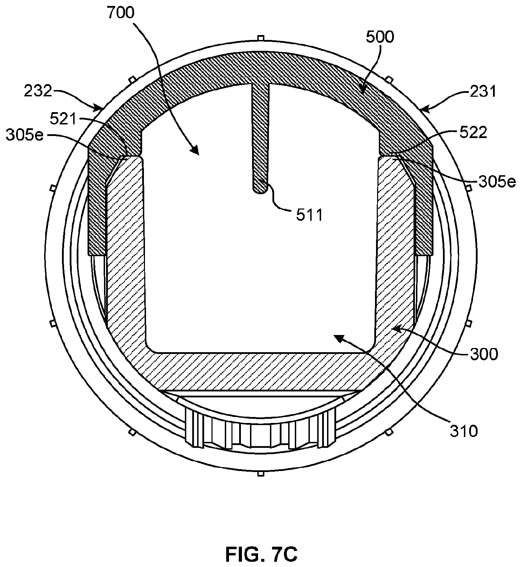

Referring to FIG. 2B, the handle assembly 200 further includes a slide chassis 300 disposed within the handle housing 230 (e.g., within the first housing portion 231 and the second housing portion 232), a first slide member 400L drivingly coupled with the first deflection wire (e.g., 131), a second slide member 400R drivingly coupled with the second deflection wire (e.g., 132), and a slide block divider 500. The slide block divider 500 is disposed within the handle housing 230 (e.g., within the first housing portion 231 and the second housing portion 232). The slide block divider 500 and the slide chassis 300 combine to form a slide compartment 700 (shown in FIG. 7C) in which each of the first slide member 400L and the second slide member 400R is slidably disposed. The slide block divider 500 is configured in part to prevent the first deflection wire (e.g., 131) and the second deflection wire (e.g., 132) from crossing each other within the handle housing 230. The slide block divider 500 and the slide chassis 300 are also configured to constrain each of the first slide member 400L and the second slide member 400R during translation within the slide compartment to inhibit motion of the first slide member 400L and the second slide member 400R transverse to a longitudinal direction of the slide compartment 700. As illustrated in FIG. 7C, the slide compartment 700 is formed between a lumen portion of the slide chassis 300 and the slide block divider 500. The lumen portion of the slide chassis 300 is a portion of a lumen 310 between a distal end 301 and a proximal end 302 of the slide chassis 300, as shown in FIG. 4A.

Referring back to FIGS. 2A-2B, the actuation assembly 220 is drivingly coupled with each of the first slide member 400L and the second slide member 400R. The actuation assembly 220, when rotated in a first direction, simultaneously drives the first slide member 400L distally and the second slide member 400R proximally within the slide compartment 700. The actuation assembly 220, when rotated in a second direction opposite to the first direction, simultaneously drives the first slide member 400L proximally and the second slide member 400R distally within the slide compartment 700.

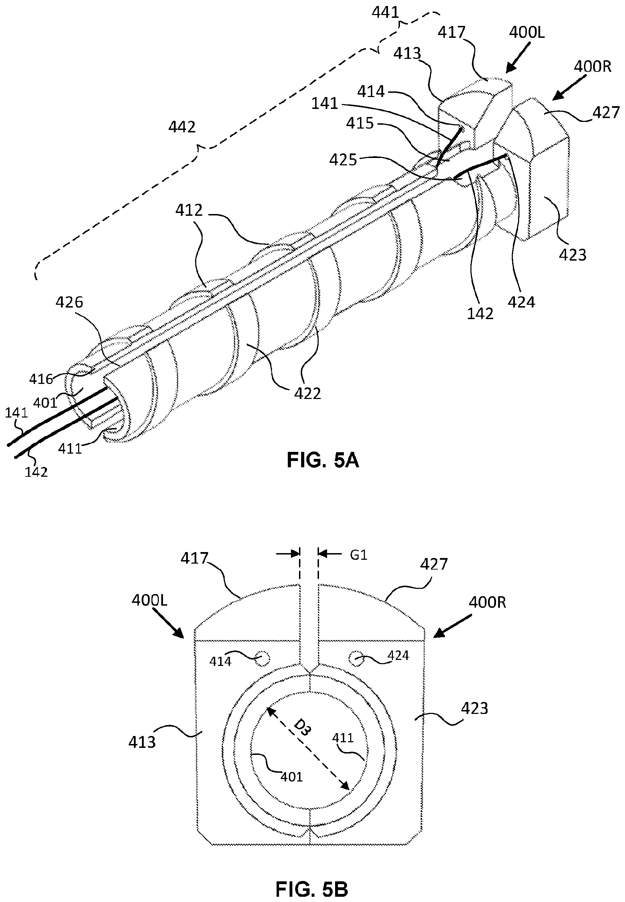

The rotatable knob 221 is coupled with the actuation hub 450. The actuation hub 450 includes internal threads 452 configured to engage the external right-handed thread and the external left-handed thread of the slide members (e.g., threads 412 and 422 in FIG. 5A). For example, the first slide member 400L may include an external right-handed thread (e.g., 412 in FIG. 5A), and the second slide member 400R may include an external left-handed thread (e.g., 422 in FIG. 5A). Accordingly, upon rotating the rotatable knob 221, the internal threads 452 engaged with the external right-handed thread and the external left-handed thread to cause the slide members 400L and 400R to move in opposite direction along the longitudinal axis of the handle housing 230.

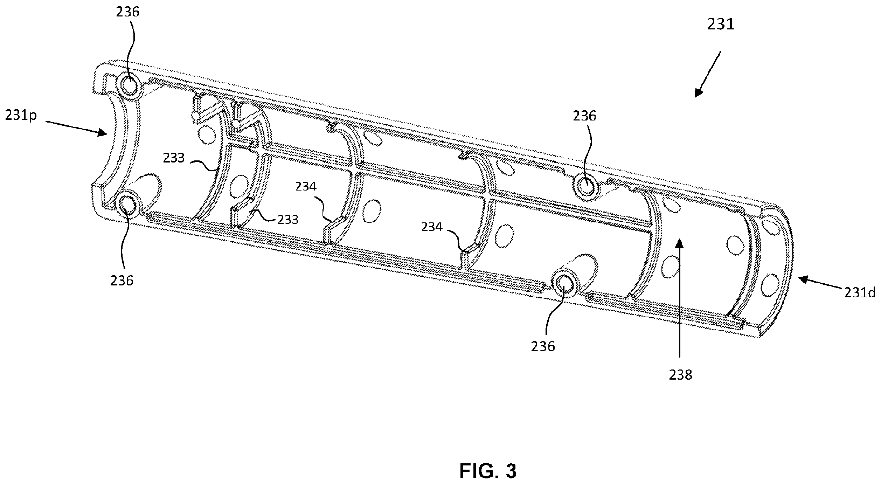

FIG. 3 shows example internal structure of the handle housing portion 231 of the handle housing 230. The first handle housing portion 231 and the second housing portion 232 may have the same internal structure, and/or include cooperating features to facilitate assembling of the handle housing portions 231 and 232. For example, an inner surface 238 of the handle housing 230 may have a curved shape with one or more ribs spaced from each other and extending along the curved shape. In some embodiments, the handle housing 230 may be substantially cylindrical in shape or a tapered cylindrical shape to provide ease of handling with one hand.

As shown in FIG. 3, the first handle housing portion 231 has a proximal end 231p and a distal end 231d. The outer diameter of the first handle housing portion 231 reduces from the proximal end 231p to the distal end 231d in a tapered manner. The inner surface 238 of the first handle housing portion 231 includes a plurality of ribs 233 spaced from each other and having partially circular shape. The ribs 233 form approximately ring or circular shape upon assembling the handle housing portions 231 and 232. The ribs 233 enhance structural integrity and restrict or prevent relative motion with other components (e.g., the slide block divider 500). One or more ribs 233 may include a radially projecting portion rib 234 to support another component (e.g., the slide chassis) and/or restrict or prevent relative motion with another component (e.g., the slide chassis 300). In some embodiments, the radially projecting rib portions 234 facilitate snug fitting of components within the handle housing 230. For example, the cross-section view in FIG. 8 illustrates the ribs 233 and the radially projecting rib portions 234 engaged with the slide block divider 500 and the slide chassis 300.

As shown in FIG. 8, a top surface of the slide block divider 500 is engaged with the ribs 233 on the inner surface of the first housing portion 231 to prevent relative motion between the handle housing 230 and the slide block divider 500. A bottom portion of the slide chassis 300 is engaged with the radially projecting rib portions 234 to support and prevent relative motion of the slide chassis 300 within the handle housing 230.

Referring back to FIG. 3, the first handle housing portion 231 may be coupled to the second handle housing portion 232 by known fastening means such as threaded screws, rivets, adhesive, or other means. Accordingly, the first handle housing portion 231 and the second handle housing portion 232 may include features for fastening. For example, the first handle housing portion 231 may include holes 236 that may be aligned with corresponding holes of the second handle housing portion 232 and a screw may be passed through the holes 236 to engage with the second handle housing portion 232.

FIGS. 4A and 4B show top side, left side, and bottom side structural aspects of the slide chassis 300. FIG. 8 is a cross-sectional view of the handle assembly 200 along a longitudinal axis of the handle assembly 200. The slide chassis 300 includes a distal end 301 and a proximal end 302. The slide chassis 300 has a length L1 and a diameter D1 that are sized to fit within the handle housing 230. The slide chassis 300 has a substantially cylindrical shape with a first diameter for the length L1, and a smaller second diameter of a stepped portion 312 at the proximal end 302.

The slide chassis 300 includes external features that engage with internal features of the handle housing 230. For example, the slide chassis 300 includes external features that engage with an inner diameter of the handle housing 230, the ribs 233, and/or the radially projecting portions 234. FIG. 4B illustrates a bottom surface 321 of the slide chassis 300 configured to engage with features on the inner surface of the handle housing 230. For example, the bottom surface 321 includes flat portions with partially circular projecting portions 323 spaced to engage with one or more of the radially projecting portions 234 or ribs 233 (e.g., see FIG. 8). The bottom portions of the bottom surface 321 rest on top of the radially projecting portions 234. As shown in FIG. 8, lumens or hollow portions of the introducer, the handle housing, and the slide members are axial aligned with each other including components such as the retaining nut 210, the actuation hub 450, and the connector 208.

The slide chassis 300 has a stepped cylindrical configuration (e.g., a hollow cylindrical shape that includes stepped portions). The slide chassis 300 has a lumen portion 310 that accommodate the slide members 400L and 400R. The lumen 310 extends between the proximal end 302 and the distal end 301. The diameter of the lumen 310 varies along the length of the slide chassis 300. The diameter of the lumen 310 is sized to accommodate at least the introducer 100 of a desired diameter (e.g., an introducer up to 15 French) and the slide members 400L and 400R. A portion of the lumen 310 has a first inner diameter and another portion of the lumen 310 has second diameter D3 at the stepped portion 312 at the proximal end 302. The first inner diameter is larger than the second diameter D3. The first inner diameter is less than diameter D1 that extends from the proximal end 302 up to the distal end 301. The first inner diameter is the diameter D1 minus a thickness of the slide chassis 300. The first inner diameter is sized to receive a distal portion of the slide members 400L and 400R. The second diameter D3 is sized to receive a selected introducer. The proximal end 302 is configured in part to limit proximal translation of the slide members 400L and 400R within the slide chassis 300.

The slide chassis 300 includes a slide member opening 305 extending along a length of the slide chassis 300. The slide member opening 305 is configured to receive and accommodate installation of the first slide member 400L and the second slide member 400R into the lumen 310 of the slide chassis 300. The slide member opening 305 is partially defined by support edges 305e. The support edges 305e are configured to support the slide block divider 500. The slide member opening 305 has a length L2 extending from the stepped portion 312 toward the distal end 301.

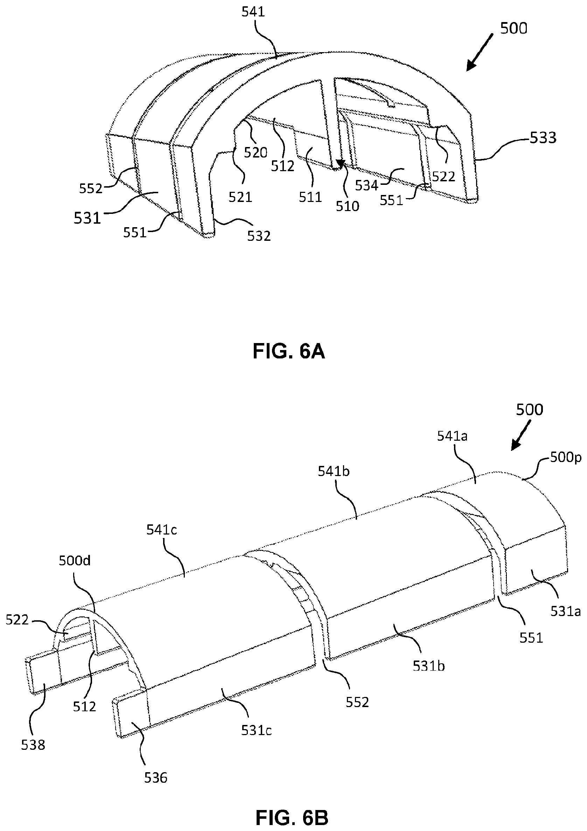

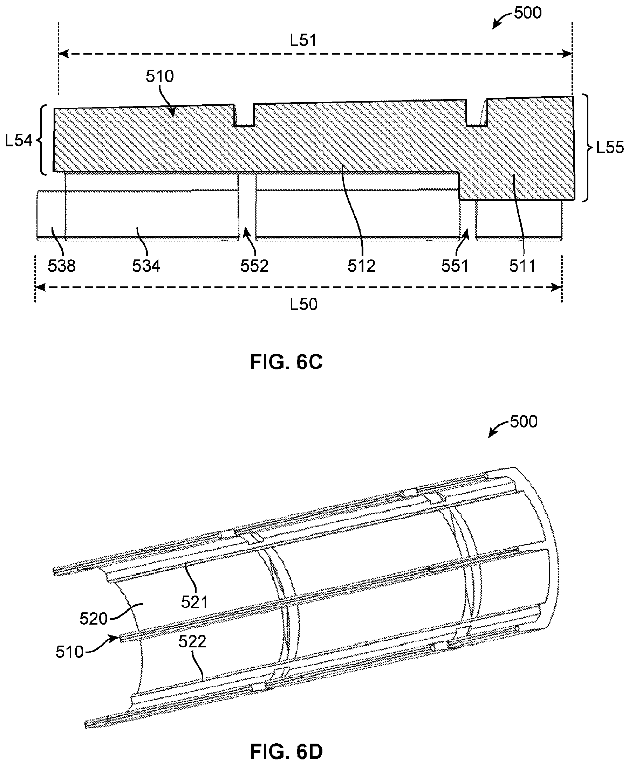

The slide chassis 300 includes a shoulder 311 for locating the slide block divider 500 within the handle assembly 200. The shoulder 311 forms a step along the stepped cylindrical body of the slide chassis 300. The shoulder 311 extends in a plane perpendicular to the support edges 305e. The slide chassis 300 includes flat side portions 313 that extend partially along a length L1 of the slide chassis 300. Inner surfaces of side walls (e.g., 531 and 533 in FIG. 6A)) of the slide block divider 500 contact the flat side portions 313. Although FIGS. 4A-4B show shoulder 311 and the flat side portion 313 on one side (e.g., a left side), the same shoulder 311 and the flat side portion 313 are included on the other side (e.g., a right side).

FIGS. 5A-5B show the slide members 400L and 400R. The slide members 400L and 400R may be mirror images of each other, each having a proximal portion 441 and a distal portion 442. The distal portion 442 of the first slide member 400L has an external right-handed thread 412. The distal portion 442 of the second slide member 400R has an external left-handed thread 422. The external right-handed thread 412 and the external left-handed thread 422 engage with the corresponding threads 452 of the actuation assembly 220 (see FIG. 2B) to cause the slide members 400L and 400R to slide axially in opposite directions when the rotatable knob 221 is rotated.

Each proximal portions 441 of the slide members 400L and 400R includes a block (e.g., a first block 413 and a second block 423) configured to anchor a deflection wire (e.g., 141 and 142). The blocks 413 and 423 are diametrically larger in size than a diameter of the distal portions 442. The blocks 413 and 423 include first curved surfaces 417 and 427 that engage corresponding inner curved surface of the handle housing 230. The blocks 413 and 423 have substantially rectangular shaped portions and include holes 414 and 424 to receive the deflection wires 141 and 142 from one side that can be coupled by retention screws 431 and 432 (see FIG. 2B) from other side of the blocks 413 and 423. The deflection wires 141 and 142 may only transmit tension forces. The deflection wires 141 and 142 may deflect or flex within an open area 415 and 425 (e.g., a cutout) formed in the distal portion 442 immediately adjacent to the proximal portion 441 of each slide members 400L and 400R when the slide member 400L or 400R displace distally.

The first slide member 400L includes a first channel 401 and the second slide member 400R includes a second channel 411. When the slide members 400L and 400R are assembled, as shown in FIG. 5B, the channels 401 and 411 combine to form the diameter D3 configured to receive the introducer 100 while allowing the slide members 400L and 400R to slide proximally and/or distally within the slide chassis 300. The deflection wires 141 and 142 extend through the channels 401 and 411. The deflection wires 141 and 142 extend from the extreme distal end of the introducer 100, through the introducer 100, and the channels 401 and 411 formed between the two slide members 400L and 400R, to a point near the proximal portion of the slide members. Each deflection wire may then be affixed to an individual slide 400L and 400R via the retention screw 431 and 432, respectively.

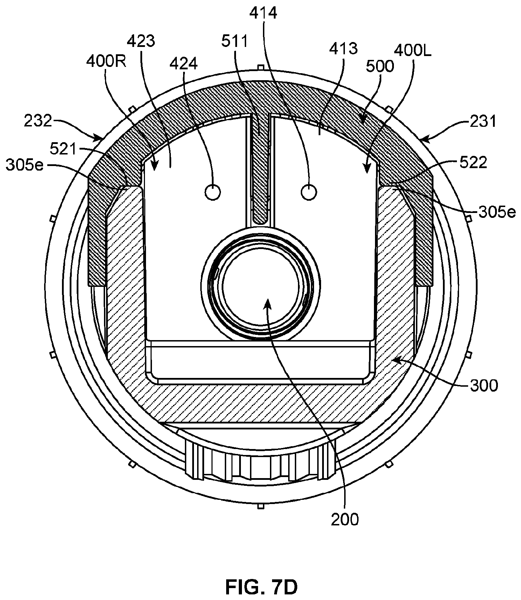

The slide members 400L and 400R include distal portion side surfaces (e.g., 416) configured to slide along either sides of a portion of the slide block divider 500 to inhibit rotation of the slide members within the slide compartment 700. The first slide member 400L includes a first slide member distal end side surface 416 configured to slide along first slide member side surfaces (e.g., a left side of a fin portion 512 shown in FIGS. 6A and 7D) of the slide block divider 500 to inhibit rotation of the first slide member 400L within the slide compartment 700. Similarly, the second slide member 400R includes second slide member distal end side surface 426 configured to slide along second slide member side surfaces (e.g., a right side of the fin portion 512 shown in FIGS. 6A and 7D) of the slide block divider 500 to inhibit rotation of the second slide member 400R within the slide compartment 700.

Referring to FIGS. 5B and 7D, the first slide member 400L and the second slide member 400R are separated by a gap G1 in which a portion of the slide block divider (e.g., 511) extends. The slide block divider 500 has an extended portion 511 at a proximal end of the slide block divider 500. The extended portion 511 is received in the gap G1 to prevent the first deflection wire 141 from crossing into the second deflection wire 142 during operation of the introducer 100. An example construction of the slide block divider 500 is further discussed with respect to FIGS. 6A-6F.

FIGS. 6A-6F illustrate different views of the slide block divider 500. The slide block divider 500 includes a proximal end 500p and a distal end 500d. The slide block divider 500 has a total length L50 (see FIG. 6C), which may be approximately equal to a length L2 of the opening 305 (in FIG. 4A) of the slide chassis 300. The slide block divider 500 may be made of plastic, metal or other material and may be removably coupled (e.g., snap fit) to the slide chassis 300 over the opening 305.

The slide block divider 500 includes a fin 510 on an inner surface 520 extending from a proximal end to a distal end of the slide block divider 500. The fin 510 is a radially projecting portion that extends from the proximal end to the distal end of the slide block divider and is disposed between the first slide member 400L and the second slide member 400R. The fin 510 prevents the slide members 400L and 400R from rotating and prevent the first deflection wire 141 from crossing with the second deflection wire 142 within the handle assembly 200.

The fin 510 of the slide block divider 500 includes a first extended portion 511 at the proximal end 500p (which has a radial length L55) and a second fin portion 512. The radial length L55 is less than a radial length L54 of the second fin portion 512. An axial length of the extended portion 511 may be approximately same as an axial length of the blocks 413 and 423 of the slide members 400L, 400R. The axial length of the first extended portion 511 may be less than the second fin portion 512. The axial length of the second fin portion 512 may be approximately same as an axial length of the distal portion 442 of the slide members 400L, 400R.

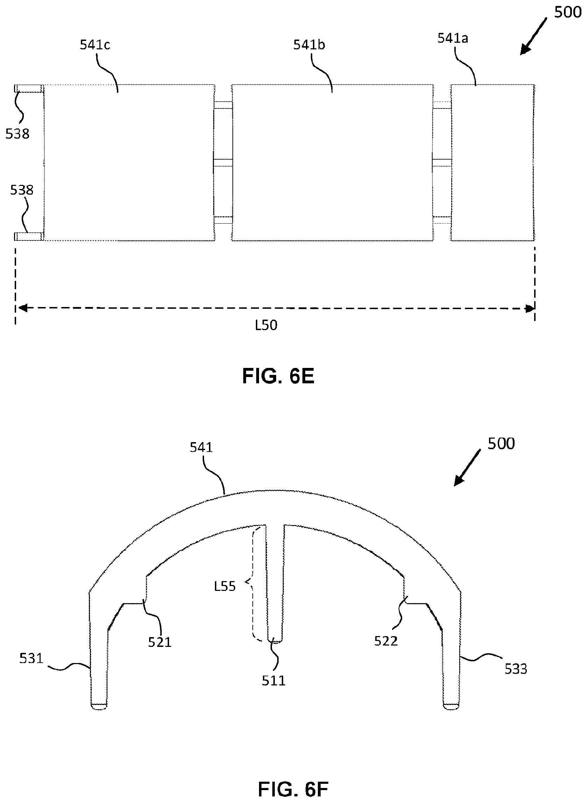

The slide block divider 500 includes an external surface 541 configured to interface with the inner surface 238 of the handle housing 230. The external surface 541 of the slide block divider 500 has a curved shape that is shaped complementary to the inner surface 238. The external surface 541 includes a first portion 541a, a second portion 541b, and a third portion 541c (see FIG. 6B and 6E). Each portion 541a, 541b, and 541c of the external surface 541 is spaced from each other via slots 551, 552. The slide block divider 500 may be an integral component manufactured as a single piece (e.g., by a molding process) or may be manufactured by fixedly joining different pieced herein.

Each of the slots 551, 552 is configured to receive one of the ribs 233 of the handle housing 230. As shown in FIG. 8, the slots 551, 552 engage with the ribs 233 to prevent relative motion of the slide block divider 500 with respect to the handle housing 230.

The slide block divider 500 includes a pair of rails 521, 522. Each of the pair of rails 521, 522 extends along a length of the slide block divider 500 and is configured to rest on the slide chassis 300 (e.g., see FIG. 7D). The slide block divider 500 includes side walls 531, 533 that extend along a length of the slide block divider 500. Each of the pair of rails 521, 522 is formed on a respective one of the side walls 531, 533. The side walls 531 and 533 have inner side surfaces 532 and 534, respectively. Each of the side walls 531, 533 include a first side wall portion 531a extending from the first portion 541a, a second side wall portion 531b extending from the second portion 541b, and a third side wall portion 531c extending from the third portion 541c (see FIG. 6B).

The side walls 531, 533 of the slide block divider 500 includes a tail portion 536, 538 configured to butt against a shoulder (e.g., 311) of the slide chassis 300 and facilitate assembly of the slide block divider 500 and the slide chassis 300. When butted with the shoulder 311 of the slide chassis 300, the tail portion 536, 538 restricts or prevents motion of the slide block divider 500 relative to the handle housing 230.

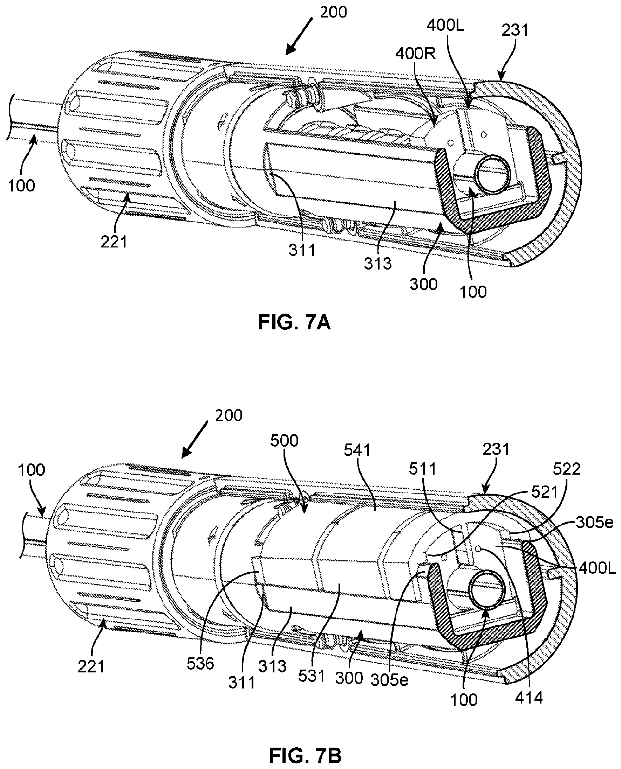

FIG. 7A shows a cutaway view of the handle assembly 200 with the slide block divider 500 removed. The rotatable knob 221 is rotatably coupled with the slide chassis 300. The slide members 400L, 400R are disposed within the lumen portion 310 of the slide chassis 300. The rotatable knob 221 is drivingly coupled to the slide members 400L, 400R via the external threads of the slide members 400L, 400R and internal threads within the rotatable knob 221 as described herein. The introducer 100 extends partially through the channel formed by the channels 401, 411 (shown in FIGS. 5A-5B) of the slide members 400L, 400R.

As shown in FIG. 7B, 7C and 7D, the slide block divider 500 is disposed over the slide chassis 300 forming the slide compartment 700 (in FIG. 7C). The inner surfaces of the side walls 531, 533 of the slide block divider 500 engage with the flat surfaces of the slide chassis 300. The rails 521, 522 rest on the support edges 305e. The extended portion 511 of the fin 510 is disposed between the slide members 400L, 400R.

When the slide members 400L and 400R move axially, the extended portion 511 of the fin 510 prevents the deflection wires 141, 142 from crossing each other. The movement of the slide members 400L, 400R articulates the deflection wires 141, 142, thereby causing the introducer 100 to deflect at the distal end 102 (in FIG. 1). The slide block divider 500 constrains the slide members 400L, 400R to linear translation within the slide chassis 300. The curved inner surface of the slide block divider 500 constrains the blocks 413, 423 from raising radially (e.g., upward in FIG. 7D).

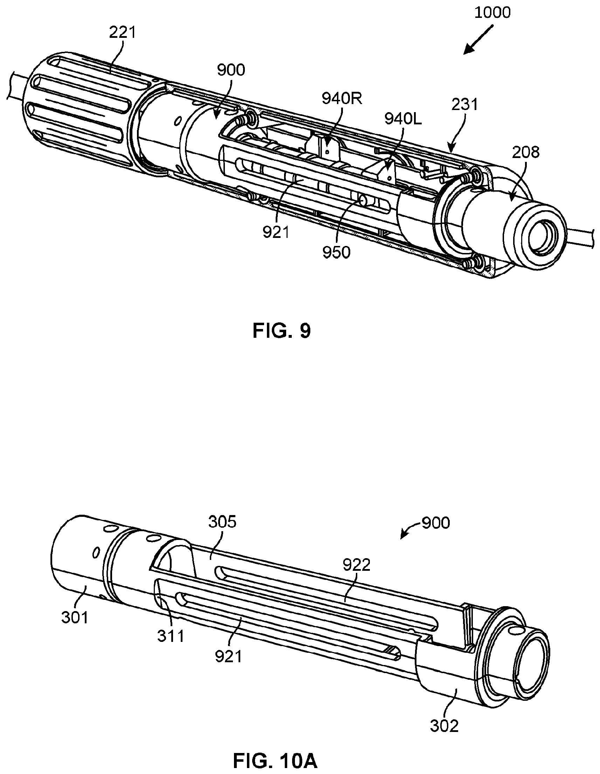

FIG. 9 shows a cutaway view of a handle assembly 1000 that is configured similar to the handle assembly 200 except for as described herein. The handle assembly 1000 includes a slide chassis 900, slide members 940L, 940R, and pins 950 (one hidden from view in FIG. 9).

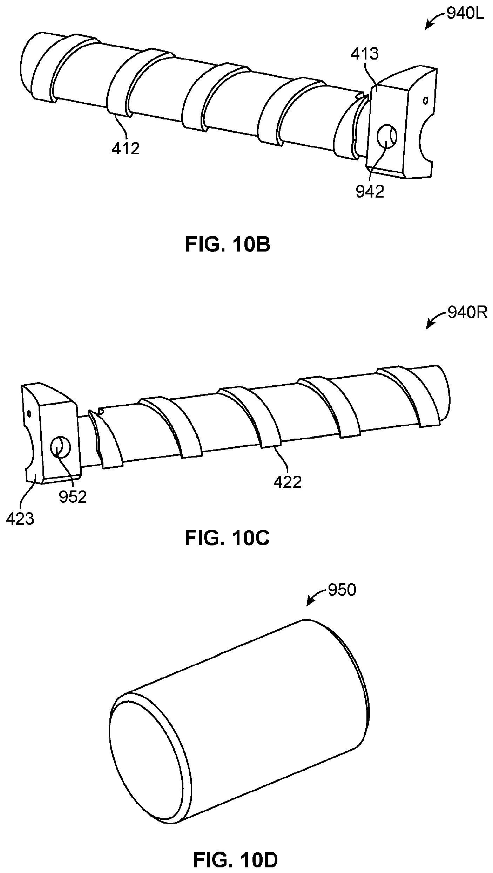

FIG. 10A shows the slide chassis 900. FIG. 10B shows the slide member 940L. FIG. 10C shows the slide member 940R. FIG. 10D shows one of the pins 950. In FIGS. 9-10D, components included in both the handle assembly 200 and the handle assembly 1000 are designated by the same reference numbers.

The slide chassis 900 of the handle assembly 1000 is configured similar to the slide chassis 300 of the handle assembly 200. The slide chassis 900 has a stepped cylindrical configuration with an opening 305 the same as the opening 305 of the slide chassis 300. The slide chassis 900 also includes a distal end 301 and the proximal end 302 with a stepped portion extending from the proximal end 302. In contrast to the slide chassis 300, the slide chassis 900 includes pin slots 921, 922 that extend at least partially along a length of the flat portions of the slide chassis 900. The length of the pin slots accommodates a suitable range of motion of the slide members 940L, 940R. Each of the pin slots 921, 922 is configured to receive one of the pins 950 and accommodate sliding of the pin 950 along the pin slot 921, 922.

The slide members 940L, 940R are configured similar to the slide members 400L, 400R of the handle assembly 200. For example, the slide member 940L includes the external thread 412 and the slide member 940R includes the external thread 422. The external threads 412, 422 are engaged with the internal threads of the rotation knob 221 to facilitate axial movement of the slide members 940L, 940R in opposite directions. Furthermore, the slide members 940L, 940R include proximal portions having the blocks 413, 423. The blocks 413, 423 include pin holes 942, 952, respectively, to receive one of the pins 950. Each of the two pins 950 extends into one of the pin holes 942, 952, which hold the pin 950 in a fixed position and orientation relative to the respective slide member 940L, 940R.

As shown in FIG. 9, the first slide member 900L is slidingly disposed within a lumen of the slide chassis 900 such that one of the pins 950 is slidably engaged within the pin slot 921. Similarly, the second slide member 900R is slidingly disposed within the lumen of the slide chassis 900 such that the second pin 950 (hidden from view in FIG. 9) is slidably engaged within the slot 922 (shown in FIG. 10A). The pins 950 and the slots 921, 922 constrain the slide members 900L, 900R transversely while allowing the slide members 900L, 900R to move longitudinally within the slide chassis 900. The pins 950 and the slots 921, 922 prevent the slide members 900L, 900R from moving radially (e.g., upward) or displacing sideways relative to the slide chassis 900. In some embodiments, the handle assembly 1000 further includes the slide block divider 500, which can be configured to be disposed over the slide chassis 900. In some embodiments, the handle assembly 1000 does not include the slide block divider 500.

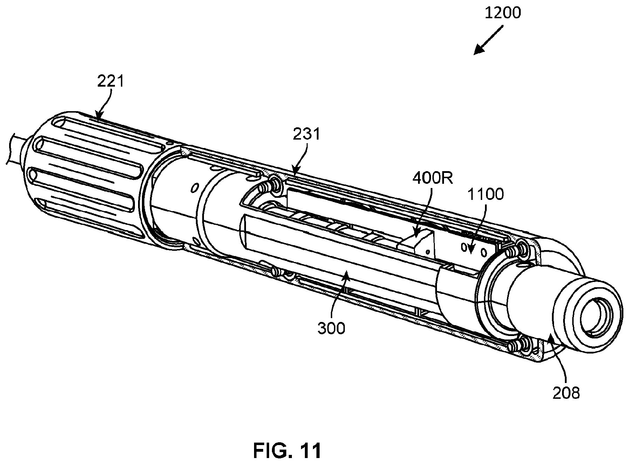

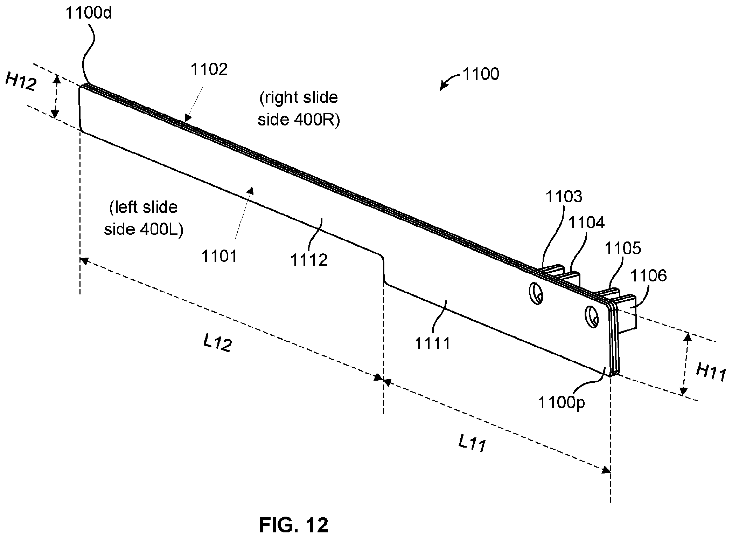

FIG. 11 shows a cutaway view of a handle assembly 1200 that is configured similar to the handle assemblies 200, 900 except for as described herein. The handle assembly 1200 includes the slide members 400L, 400R and a slide block divider 1100. As shown in FIG. 12, the slide block divider 1100 includes an elongated profiled plate having a distal end 1100d, a proximal end 1100p, a first face surface 1101 on one side, and a second face surface 1102 on other opposite side. The elongated profiled plate of the slide block divider 1100 is similar in shape and function to the fin 510 of the slide block divider 500 (e.g., see FIG. 6C). The slide block divider 1100 includes a first portion 1111 and a second portion 1112 that have shapes that are complementary to the external profiles of the slide members 400L, 400R. The first portion 1111 has a first length L11 corresponding to a length of the proximal portion 441 of the slide members 400L, 400R (see FIG. 5A) and a first height H11 corresponding to a distance between the inner surface 238 of the handle housing 230 to a top of the slide members 400L, 400R. The second portion 1112 has a second length L12 corresponding to a length of the distal portion 442 of the slide members 400L, 400R (see FIG. 5A) and a second height H12 corresponding to a depth of the gap (e.g., G1 in FIG. 5B) between the slide members 400L, 400R.

In the illustrated embodiment, the second face surface 1102 includes lateral projections 1103, 1104, 1105, 1106 that protrude perpendicular to and from the second face surface 1102. The lateral projections 1103, 1104, 1105, 1106, however, may be provided at the first portion 1111 and protrude perpendicular to and from the first face surface 1101. The lateral projections 1103, 1104, 1105, 1106 may be sized and spaced corresponding to the ribs 233 of the handle housing 230 (in FIG. 3) and may be engaged with the ribs 233 to prevent relative motion between the slide block divider 1100 and the handle housing 230.

The slide block divider 1100 is disposed between portions of the slide members 400L, 400R. The second portion 1112 is located between the distal portions 442 of the slide members 400L, 400R. The second portion 1112 constrains the slide members 400L, 400R (e.g., in upward or sideways direction relative to the slide chassis 300) while allowing axial motion of the slide members 400L, 400R within the slide chassis 300. The first slide member side surface of the slide member 400L slides along the first face surface 1101 and the second slide member side surfaces of the other slide member 400R slides along the second face surface 1102. The first portion 1111 extends between the gap (e.g., G1 in FIG. 5B) and prevents the deflection wires 141, 142 from crossing each other while the slide members 400L, 400R slide in opposite directions within the slide chassis 300.

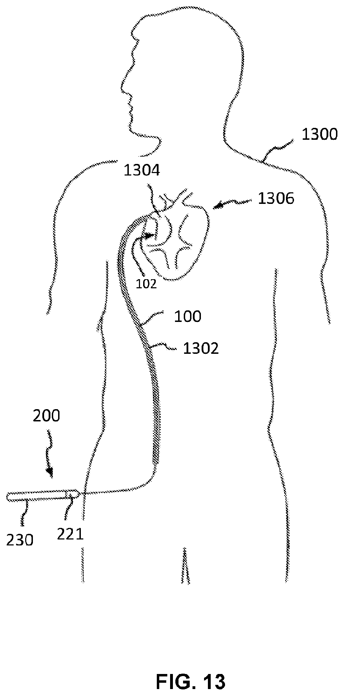

FIG. 13 is a diagrammatic illustration of the introducer 100 employed in a procedure on a patient 1300. The distal end 102 of the introducer 100 is inserted into the patient 1300 (e.g., intravenously via a body lumen 1302 of the patient 1300, percutaneously, or via other avenues for entering the patient's body). The distal end 102 of the introducer 100 is advanced until positioned in a selected location within the patient 1300 (e.g., within a chamber 1304 of the patient's heart 1306 or other organ, with a body cavity of the patient, etc.). The distal end of the introducer 100 is then deflected by rotating the adjustment knob 221 about a longitudinal axis of the handle housing 230. As can be understood from FIGS. 1-12, this causes the slide members 400L, 400R within the handle housing 230 to displace along the longitudinal axis in opposite directions. Since each of the slide members 400L, 400R is coupled to its respective deflection wire 141, 142 and each deflection wire 141,142 runs through the introducer 100 and is coupled to the distal end 102, the distal end 102 of the introducer 100 is deflected.

The introducer 100 can be used in conjunction with any suitable catheter, such as a diagnostic catheter, an RF ablation catheter, an electroporation catheter, etc. Example electroporation systems and catheters for electroporation systems are discussed in a PCT publication no. WO 2018102376A1.

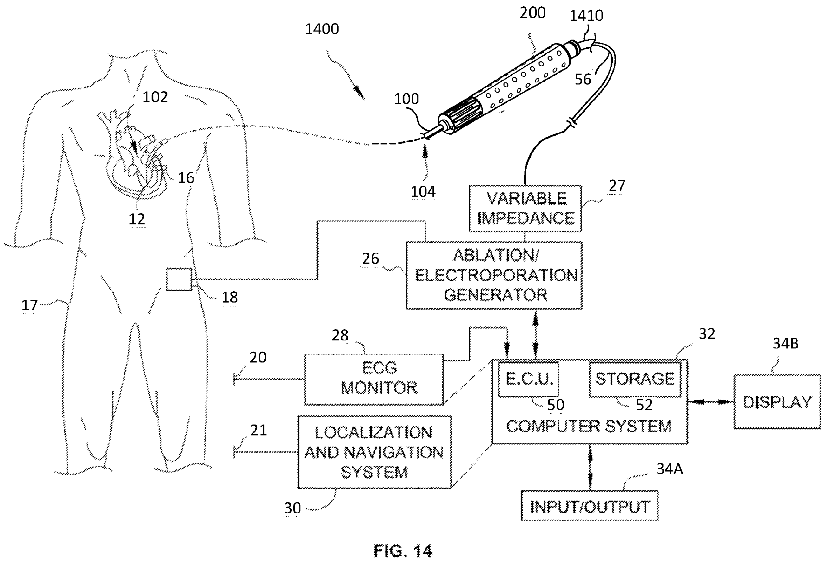

FIG. 14 is a diagrammatic and block diagram view of a system 1400 for electroporation therapy employing the handle assembly 200 of the introducer 100. The system 1400 may be used for irreversible electroporation to destroy tissue. In particular, the system 1400 may be used for electroporation-induced primary necrosis therapy, which refers to the effects of delivering electrical current in such manner as to directly cause an irreversible loss of plasma membrane (cell wall) integrity leading to its breakdown and cell necrosis. This mechanism of cell death may be viewed as an "outside-in" process, meaning that the disruption of the outside wall of the cell causes detrimental effects to the inside of the cell. Typically, for classical plasma membrane electroporation, electric current is delivered as a pulsed electric field (*i.e.,* pulsed field ablation (PFA)) in the form of short-duration pulses (e.g., 0.1 to 20 ms duration) between closely spaced electrodes capable of delivering an electric field strength of about 0.1 to 1.0 kV/cm.

The handle assembly 200, 900, 1200 provides a location for the clinician to hold the introducer 100 and may further provide means for steering or the guiding introducer shaft 110 within body 17. For example, the handle assembly 200, 900, 1200 may include means to change the length of a guidewire extending through introducer 100 to the distal end 102. Moreover, in some embodiments, the handle assembly 200, 900, 1200 may be configured to vary the shape, size, and/or orientation of a portion of the introducer 100. The distal end 102 of the introducer 100 may be advanced until positioned in a selected location within the patient body 17. The distal end 102 of the introducer shaft 110 is then deflected by rotating the adjustment knob 221 about a longitudinal axis of the handle assembly 200, 900, 1200. As can be understood from FIGS. 1-12, this causes the slide members 400L, 400R within the handle housing 230 to displace along the longitudinal axis in opposite directions. Since each of the slide members 400L, 400R is coupled to its respective deflection wire 141, 142 and each deflection wire 141, 142 runs through the introducer 100 and is coupled to the distal end 102, the distal end 102 of the introducer 100 is deflected.

The introducer 100 can be used to introduce a catheter 1410 (e.g., a PFA catheter) into the heart (e.g., right or left atrium) to perform electroporation ablation to correct conditions such as cardiac arrhythmias. Once the introducer 100 is positioned at the selected location, the catheter 1410 may be introduced through the proximal end of the introducer 100. For example, the catheter 1410 may be inserted into a lumen of the introducer shaft 110 from the proximal end of the introducer 100 and slowly advanced through the lumen until a tip of the catheter 1410 extends beyond the distal end 102 of the introducer 100 and is positioned at the selected location. The catheter 1410 may include electrode(s) 12 used for a variety of diagnostic and therapeutic purposes including, for example and without limitation, cardiac mapping and/or ablation (e.g., electroporation ablation). For example, and in some embodiments, the electrode(s) may be configured as a bipolar electrode assembly for use in bipolar-based electroporation therapy. Specifically, the electrode(s) 12 can be individually electrically coupled to generator 26 (e.g., via suitable electrical cable 56 or other suitable electrical conductors extending through a proximal end of the catheter 1410) and are configured to be selectively energized (e.g., by an electroporation generator 26 and/or computer system 32) with opposite polarities to generate a potential and corresponding electric field therebetween, for electroporation therapy. That is, one of electrode(s) 12 is configured to function as a cathode, and the other is configured to function as an anode. Electrode(s) 12 may be any suitable electroporation electrodes. In the exemplary embodiment, electrode(s) 12 are ring electrodes. Electrode(s) 12 may have any other shape or configuration. It is realized that the shape, size, and/or configuration of electrode(s) 12 may impact various parameters of the applied electroporation therapy. For example, increasing the surface area of one or both electrode(s) 12 may reduce the applied voltage needed to cause the same level of tissue destruction. While the electrode(s) 12 is described as a bipolar electrode, it should be understood that in some embodiments, electrode(s) may be configured as a monopolar electrode and use a patch electrode (e.g., return electrode 18) as a return or indifferent electrode.

In some embodiments, a plurality of return electrodes 18, 20, and 21, which are diagrammatic of the body connections may be used by the various sub-systems included in the overall system 1400, such as the electroporation generator 26, an electrophysiology (EP) monitor such as an ECG monitor 28, or a localization and navigation system 30 for visualization, mapping and navigation of internal body structures. In the illustrated embodiment, return electrodes 18, 20, and 21 are patch electrodes. It should be understood that the illustration of a single patch electrode is diagrammatic only (for clarity) and that such sub-systems to which these patch electrodes are connected may, and typically will, include more than one patch (body surface) electrode. The system 1400 may further include a main computer system 32 (including an electronic control unit 50 and data storage-memory 52), which may be integrated with system 30 in certain embodiments. The computer system 32 may further include conventional interface components, such as various user input/output mechanisms 34a and a display 34b, among other components.

The electroporation generator 26 may be configured to energize the electrode element(s) in accordance with an electroporation energization strategy, which may be predetermined or may be user-selectable. A variable impedance 27 allows the impedance of the system to be varied to limit arcing from the electrode(s) 12 of the catheter 1410. Moreover, variable impedance 27 may be used to change one or more characteristics, such as amplitude, duration, pulse shape, and the like, of an output of electroporation generator 26. Although illustrated as a separate component, variable impedance 27 may be incorporated in the catheter 1410 or generator 26. In some embodiments, each variable impedance 27 may be connected to a different catheter electrode or group of catheter electrodes to allow the impedance through each catheter electrode or group of catheter electrodes to be separately varied.

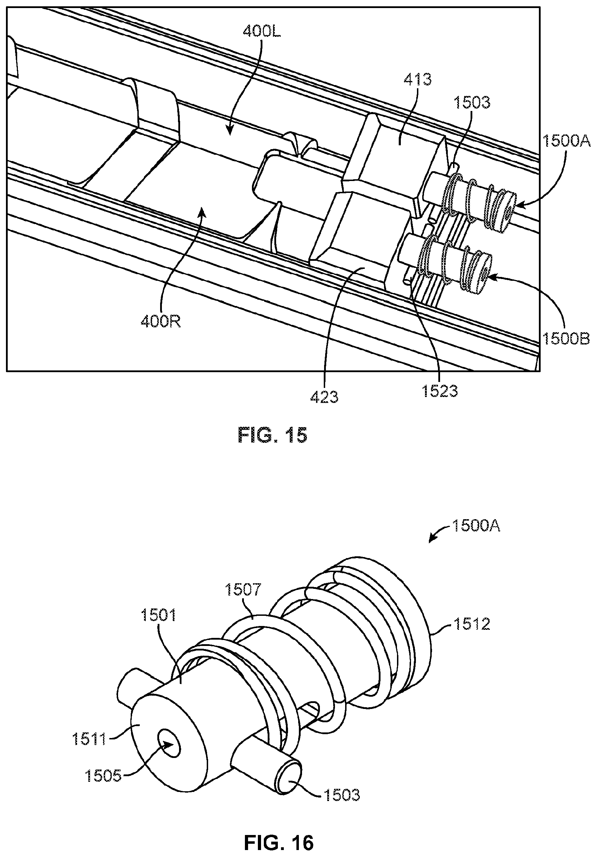

FIG. 15 and FIG. 16 illustrate pin assemblies configured to attach deflection wires to slide members in a handle assembly herein. In FIG. 15, pin assemblies are coupled to the slide members 400L and 400R, respectively of the handle assembly 200 of the introducer 100 (e.g., see FIG. 1). In the illustrated embodiment, the handle assembly 200 includes a first pin assembly 1500A and a second pin assembly 1500B to couple the deflection wires (e.g., the deflection wires 141 and 142 in FIG. 5A). Each pin assembly includes a connector shaft having a wire channel, a pin, and a spring.

For example, as shown in FIG. 16, the first pin assembly 1500A includes a first connector shaft 1501, a first pin 1503, and a first spring 1507. The first connector shaft 1501 is cylindrical in shape and the first pin 1503 is transversely disposed through the first connector shaft 1501. The first spring 1507 is disposed over the first connector shaft 1501 and abuts against the first pin 1503 to secure the pin 1503 in place. The first connector shaft 1501 includes a wire channel 1505 extending from a distal end 1511 to a proximal end 1512 of the first connector shaft 1501. The wire channel 1505 is configured to axially receive a deflection wire passed through the slide member 400L. The wire channel 1505 of the first connector shaft 1501 can be aligned with a deflection wire channel (e.g., see the channel 414 in FIG. 7D) of the slide member 400L. The deflection wire (e.g., 141 in FIG. 5A) can be received through the wire channel 1505 and coupled to the first pin 1501.

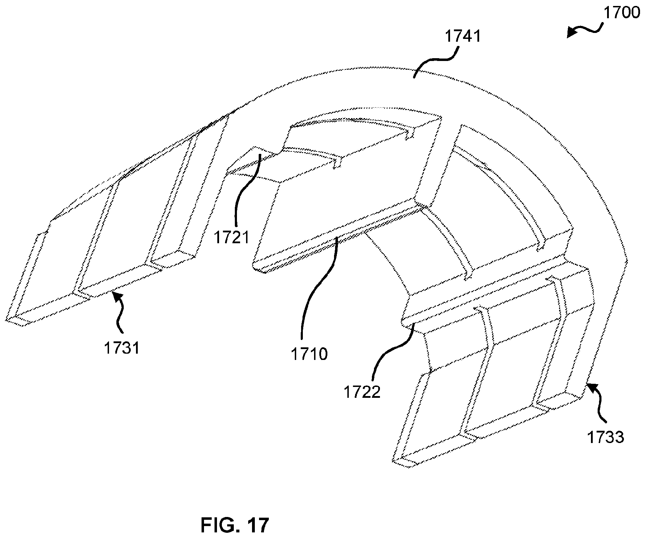

As shown in FIG. 15, the first pin 1503 of the first pin assembly 1500A is disposed at a proximal end of the first deflection wire channel (e.g., 414) of the first slide member 400L. The first connector shaft 1501 extends axially from the slide member 400L while the first pin 1502 extends transversely. Similar to the first pin assembly 1500A, the second pin assembly 1500B has a second pin 1523 disposed at a proximal end of a second deflection wire channel of the second slide member 400R. The second deflection wire (e.g., 142) is coupled to the second pin 1523. The first pin 1503 and the second pin 1523 extend toward each other and may limit a vertical height available for a fin of the slide block divider used to prevent the deflection wires from crossing each other when the slide members 400L and 400R move relative to each other. Hence, for the handle 200 employing the pin assemblies 1500A and 1500B, a modified slide block divider can be provided. An example slide block divider 1700 is illustrated and discussed in detail with respect to FIG. 17 through FIG. 20.

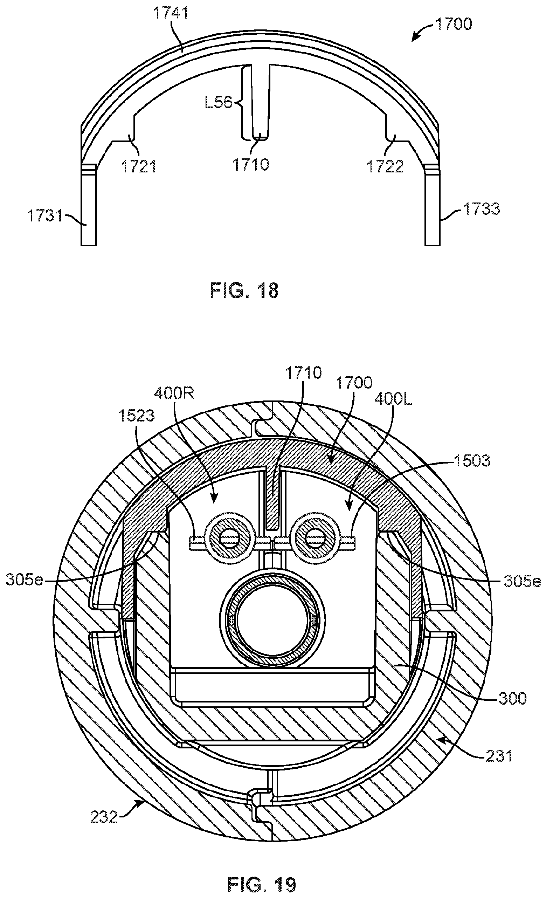

Referring to FIG. 17 through FIG. 20 construction and assembly of the slide block divider 1700 in the handle 200 is discussed. The slide block divider 1700 has similar construction as the slide block divider 500 with a modified fin. For example, the slide block divider 1700 includes a pair of rails 1721, 1722 (similar to the rails 521, 522 in FIG. 6A). Each of the pair of rails 1721, 1722 extends along a length of the slide block divider 1700 and is configured to rest on the slide chassis 300 (e.g., on the edge 305e, see FIG. 19). The slide block divider 1700 includes side walls 1731, 1733 (similar to the walls 531, 533) that extend along a length of the slide block divider 1700.

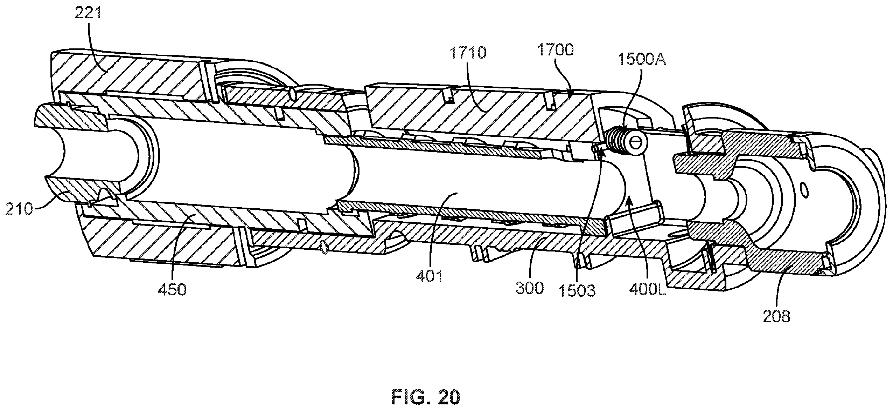

The slide block divider 1700 includes a fin 1710 extending radially toward the slide members 400L and 400R. The fin 1710 has a height L56 extending radially down to and above the first pin 1503 and the second pin 1523 (best seen in FIG. 19 and FIG. 20). Thus, the fin 1710 does not interfere with the pins 1503, 1523 during sliding of the slide members 400L, 400R. The height of the fin is approximately constant along a longitudinal axis of the slide block divider 1700 (see FIG. 17 and FIG. 20). The height L56 of the fin 1710 of the slide block divider 1700 can be less than the height L55 of the extended portion 511 of the fin 510 of the slide block divider 500 (see FIGS. 18 and 6F).

It can be understood that the present disclosure is not limited to a constant height fin, as modification of fin shape along the length of the fin as possible. For example, the fin may have a tapered shape to prevent the first pin 1503 and the second pin 1523 from coming in contact with the fin during sliding of the slide members 400L and 400R. For example, at the pins 1503, 1523 the fin has a shorter height, and the height gradually increases along the length without interfering with the slide members.

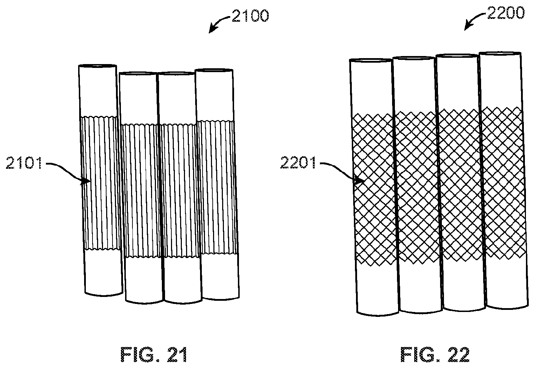

Furthermore, the pins 1503 and 1523 can be modified to include textured circumferential surface, for example, to provide improved gripping between the deflection wire and the pin. For example, in FIG. 21, the pins 2100 may include a first textured circumferential surface portion 2101. The first textured surface is serrations. As another example, in FIG. 22, pins 2200 may include a second textured circumferential surface portion 2201. The second textured surface are diamond shape texture.

It can be understood that the catheter 1410 and electrode configuration herein are presented by way of example and does not limit the scope of the present disclosure. A person of ordinary skill in the art may employ other types of catheters and/or electrodes.

In one or more embodiments of the present disclosure, a steerable introducer includes an elongated body, a first deflection wire, a second deflection wire, a handle assembly, and an actuation assembly. The elongated body includes a proximal end, a steerable section, a first deflection lumen extending between the proximal end and the steerable section, and a second deflection lumen extending between the proximal end and the steerable section. The first deflection wire extends within the first deflection lumen and actuatable to induce deflection of the steerable section of the elongated body in a first direction. The second deflection wire extends within the second deflection lumen and actuatable to induce deflection of the steerable section of the elongated body in a second direction. The handle assembly is coupled to the proximal end of the elongated body. The handle assembly include a slide chassis, a first slide member, a second slide member, and a slide block divider. The slide chassis is disposed within the handle housing. The first slide member is drivingly coupled with the first deflection wire. The second slide member drivingly coupled with the second deflection wire. The slide block divider disposed within the handle housing. The slide block divider and the slide chassis combine to: form a slide compartment in which each of the first slide member and the second slide member is slidably disposed, prevent the first deflection wire and the second deflection wire from crossing each other within the handle housing, and constrain each of the first slide member and the second slide member during translation within the slide compartment to inhibit motion transverse to a longitudinal direction of the slide compartment. The actuation assembly drivingly coupled with each of the first slide member and the second slide member. The actuation assembly is operable to simultaneously drive the first slide member distally and the second slide member proximally within the slide compartment, and wherein the actuation assembly is operable to simultaneously drive the first slide member proximally and the second slide member distally within the slide compartment. Optionally, the slide chassis includes a slide member opening extending along a length of the slide chassis. Optionally, the slide member opening is configured to accommodate installation of at least one of the first slide member and the second slide member into the slide chassis. Optionally, the slide chassis includes support edges configured to support the slide block divider. Optionally, the slide member opening is at least partially defined by the support edges. Optionally, the slide chassis includes shoulders for locating the slide block divider, and the shoulders extend in a plane perpendicular to the support edges at an end close to the distal end of the slide chassis. Optionally, the slide chassis is substantially cylindrical in shape. Optionally, the slide block divider comprises a fin extending toward the slide members to prevent the slide members from rotating and to prevent the first deflection wire from crossing with the second deflection wire within the handle housing. Optionally, the fin is a radially projecting portion that extends from a proximal end of the slide block divider to a distal end of the slide block divider and is disposed between the first slide member and the second slide member. Optionally, the handle housing comprises an inner surface. The slide block divider includes an external surface interfaced with the inner surface of the handle housing. Optionally, the inner surface of the handle housing has a curved shape with one or more ribs spaced from each other and extending along the curved shape. The external surface of the slide block divider has a corresponding curved shape with slots to receive the one or more ribs of the inner surface of the handle housing and to prevent relative motion of the slide block divider with respect to the handle. Optionally, the slide block divider comprises a pair of rails, and each of the pair of rails extends along a length of the slide block divider and is configured to rest on the slide chassis. Optionally, the slide block divider comprises side walls extending along a length of the slide block divider. Each of the pair of rails is formed on a respective one of the side walls. Optionally, the side walls of the slide block divider comprise a tail portion configured to butt against a shoulder of the slide chassis to facilitate assembly and to prevent relative motion between the slide block divider and the slide chassis. Optionally, the slide chassis comprises flat side portions extending at least partially along a length of the slide chassis, and inner surfaces of the side walls of the slide block divider contact the flat side portions. Optionally, the first slide member has an external right-handed thread. The second slide member has an external left-handed thread. The actuation assembly includes a rotatable knob having internal threads configured to engage the external right-handed thread and the external left-handed thread. Optionally, the first slide member comprises first slide member distal end side surfaces configured to slide along first slide member side surfaces of the slide block divider to inhibit rotation of the first slide member within the slide compartment. The second slide member includes second slide member distal end side surfaces configured to slide along second slide member side surfaces of the slide block divider to inhibit rotation of the second slide member within the slide compartment. Optionally, the first slide member includes a first deflection wire channel through which the first deflection wire extends, and the second slide member includes a second deflection wire channel through which the second deflection wire extends. Optionally, the steerable introducer further includes a first pin assembly, and a second pin assembly. The first pin assembly has a first pin disposed at a proximal end of the first deflection wire channel. The first deflection wire is coupled to the first pin. The second pin assembly has a second pin disposed at a proximal end of the second deflection wire channel. The second deflection wire is coupled to the second pin. Optionally, the slide block divider includes a fin extending radially toward the slide members, wherein the fin has a height extending radially down to and above the first pin and the second pin. Optionally, the height of the fin is constant along a longitudinal axis of the slide block divider. Optionally, the first slide member and the second slide member are separated by a gap in which a portion of the slide block divider extends. Optionally, the slide block divider has an extended portion at a proximal end of the slide block divider. The extended portion is received in the gap and prevents the first deflection wire from crossing into the second deflection wire during operation of the steerable introducer. Optionally, the fin has a tapered shape to prevent the first pin and the second pin from coming in contact with the fin during sliding of the slide members. Optionally, each of the first pin and the second pin comprises a textured circumferential surface.

Disjunctive language such as the phrase "at least one of X, Y, or Z," unless specifically stated otherwise, is intended to be understood within the context as used in general to present that an item, term, etc., may be either X, Y, or Z, or any combination thereof (e.g., X, Y, and/or Z). Thus, such disjunctive language is not generally intended to, and should not, imply that certain embodiments require at least one of X, at least one of Y, or at least one of Z to each be present.

While certain embodiments have been described, these embodiments have been presented by way of example only and are not intended to limit the scope of the present disclosures. Indeed, the novel methods, apparatuses and systems described herein can be embodied in a variety of other forms; furthermore, various omissions, substitutions and changes in the form of the methods, apparatuses and systems described herein can be made without departing from the scope of the present disclosures.

## Claims

1. A steerable introducer (100) comprising:
an elongated body (110) comprising a proximal end (104), a steerable section (103), a first deflection lumen (131) extending between the proximal end (104) and the steerable section (103), and a second deflection lumen (132) extending between the proximal end (104) and the steerable section (103);
a first deflection wire (141) extending within the first deflection lumen (131) and actuatable to induce deflection of the steerable section of the elongated body in a first direction;
a second deflection wire (142) extending within the second deflection lumen (132) and actuatable to induce deflection of the steerable section of the elongated body in a second direction;
a handle assembly (200) coupled to the proximal end (104) of the elongated body, the handle assembly comprising:
a handle housing (230) comprising an inner surface (238);
a slide chassis (300) disposed within the handle housing (230);
a first slide member (400L) drivingly coupled with the first deflection wire (131);
a second slide member (400R) drivingly coupled with the second deflection wire (142);
a slide block divider (500) disposed within the handle housing and comprising an external surface (541) interfaced with the inner surface (238) of the handle housing, wherein the slide block divider (500) and the slide chassis (300) combine to:
form a slide compartment (700) in which each of the first slide member (400L) and the second slide member (400R) is slidably disposed,
prevent the first deflection wire (131) and the second deflection wire (142) from crossing each other within the handle housing, and
constrain each of the first slide member (400L) and the second slide member (400R) during translation within the slide compartment (700) to inhibit motion transverse to a longitudinal direction of the slide compartment; and
an actuation assembly (220) drivingly coupled with each of the first slide member (400L) and the second slide member (400R), wherein the actuation assembly is operable to simultaneously drive the first slide member (400L) distally and the second slide member (400R) proximally within the slide compartment (700), and wherein the actuation assembly (220) is operable to simultaneously drive the first slide member (400L) proximally and the second slide member (400R) distally within the slide compartment.

2. The steerable introducer of claim 1, wherein:
the slide chassis (300) includes a slide member opening (305) extending along a length of the slide chassis; and
the slide member opening (305) is configured to accommodate installation of at least one of the first slide member (400L) and the second slide member (400R) into the slide chassis.

3. The steerable introducer of claim 2, wherein:
the slide chassis (300) comprises support edges (305e) configured to support the slide block divider (500); and
the slide member opening (305) is at least partially defined by the support edges (305e).

4. The steerable introducer of claim 3, wherein the slide chassis (300) includes shoulders (311) for locating the slide block divider (500), and the shoulders extend in a plane perpendicular to the support edges (305e) at an end close to the distal end of the slide chassis.

5. The steerable introducer of any one of claims 1 through 4, wherein the slide chassis (300) is substantially cylindrical in shape.

6. The steerable introducer of any one of claims 1 through 5, wherein the slide block divider (500, 1700) comprises a fin (510, 1710) extending toward the slide members (400L, 400R) to prevent the slide members from rotating and to prevent the first deflection wire (131) from crossing with the second deflection wire (142) within the handle housing, optionally, wherein the fin (510, 1710) is a radially projecting portion that extends from a proximal end of the slide block divider to a distal end of the slide block divider and is disposed between the first slide member (400L) and the second slide member (400R).

7. The steerable introducer of any one of claims 1 through 6, wherein:
the inner surface (238) of the handle housing has a curved shape with one or more ribs (233) spaced from each other and extending along the curved shape; and
the external surface (541) of the slide block divider has a corresponding curved shape with slots (551, 552) to receive the one or more ribs (233) of the inner surface of the handle housing and to prevent relative motion of the slide block divider (500) with respect to the handle (200).

8. The steerable introducer of any one of claims 1 through 7, wherein:
the slide block divider (500) comprises a pair of rails (521, 522); and
each of the pair of rails (521, 522) extends along a length of the slide block divider and is configured to rest on the slide chassis.

9. The steerable introducer of claim 8, wherein:
the slide block divider (500) comprises side walls (531, 533) extending along a length of the slide block divider; and
each of the pair of rails (521, 522) is formed on a respective one of the side walls (531, 533).

10. The steerable introducer of claim 9, wherein the side walls (531, 533) of the slide block divider comprise a tail portion (536, 538) configured to butt against a shoulder (311) of the slide chassis to facilitate assembly and to prevent relative motion between the slide block divider and the slide chassis, or, wherein:
the slide chassis (300) comprises flat side portions (313) extending at least partially along a length of the slide chassis; and
inner surfaces of the side walls of the slide block divider contact the flat side portions.

11. The steerable introducer of any one of claims 1 through 10, wherein
the first slide member (400L) has an external right-handed thread (412);
the second slide member (400R) has an external left-handed thread (422);
the actuation assembly comprises a rotatable knob (221) having internal threads (452) configured to engage the external right-handed thread and the external left-handed thread.

12. The steerable introducer of any one of claims 1 through 11, wherein:
the first slide member (400L) comprises first slide member distal end side surfaces (416) configured to slide along first slide member side surfaces (512) of the slide block divider to inhibit rotation of the first slide member within the slide compartment; and
the second slide member (400R) comprises second slide member distal end side surfaces (426) configured to slide along second slide member side surfaces (512) of the slide block divider to inhibit rotation of the second slide member within the slide compartment

13. The steerable introducer of any one of claims 1 through 12, wherein:
the first slide member (400L) comprises a first deflection wire channel (401) through which the first deflection wire extends; and
the second slide member (400R) comprises a second deflection wire channel (411) through which the second deflection wire extends.

14. The steerable introducer of claim 13, further comprises:
a first pin assembly (1500A) having a first pin (1503) disposed at a proximal end of the first deflection wire channel, wherein the first deflection wire is coupled to the first pin; and
a second pin assembly (1500B) having a second pin (1523) disposed at a proximal end of the second deflection wire channel, wherein the second deflection wire is coupled to the second pin.

15. The steerable introducer of claim 14, wherein the slide block divider comprises a fin (1710) extending radially toward the slide members (400L, 400R), wherein the fin has a height extending radially down to and above the first pin and the second pin, optionally, wherein the height of the fin (1710) is constant along a longitudinal axis of the slide block divider.

16. The steerable introducer of any one of claims 13 through 15, wherein the first slide member (400L) and the second slide member (400R) are separated by a gap (G1) in which a portion of the slide block divider extends, optionally,
wherein:
the slide block divider (500) has an extended portion (511) at a proximal end of the slide block divider;
the extended portion (511) is received in the gap (G1) and prevents the first deflection wire (132) from crossing into the second deflection wire (142) during operation of the steerable introducer.

## Patentansprüche

1. Lenkbare Einführvorrichtung (100), umfassend:
einen länglichen Körper (110), umfassend ein proximales Ende (104), einen lenkbaren Bereich (103), ein erstes Ablenkungslumen (131), das sich zwischen dem proximalen Ende (104) und dem lenkbaren Bereich (103) erstreckt, und ein zweites Ablenkungslumen (132), das sich zwischen dem proximalen Ende (104) und dem lenkbaren Bereich (103) erstreckt;
einen ersten Ablenkungsdraht (141), der sich innerhalb des ersten Ablenkungslumens (131) erstreckt und betätigbar ist, um eine Ablenkung des lenkbaren Bereichs des länglichen Körpers in eine erste Richtung zu bewirken;
einen zweiten Ablenkungsdraht (142), der sich innerhalb des zweiten Ablenkungslumens (132) erstreckt und betätigbar ist, um eine Ablenkung des lenkbaren Bereichs des länglichen Körpers in eine zweite Richtung zu bewirken;
eine Griffbaugruppe (200), die mit dem proximalen Ende (104) des länglichen Körpers gekoppelt ist, wobei die Griffbaugruppe Folgendes umfasst:
ein Griffgehäuse (230) umfassend eine innere Oberfläche(238);
ein Gleitchassis (300), das innerhalb des Griffgehäuses (230) angeordnet ist;
ein erstes Gleitelement (400L), das antriebsmäßig mit dem ersten Ablenkungsdraht (131) gekoppelt ist;
ein zweites Gleitelement (400R), das antriebsmäßig mit dem zweiten Ablenkungsdraht (142) gekoppelt ist;
einen Gleitblockteiler (500), der innerhalb des Griffgehäuses angeordnet ist und eine äußere Oberfläche (541) umfasst, die an die innere Oberfläche (238) des Griffgehäuses angrenzt, wobei der Gleitblockteiler(500) und das Gleitchassis (300) zu Folgendem kombiniert sind:
Bilden eines Gleitfachs (700), in dem sowohl das erste Gleitelement (400L) als auch das zweite Gleitelement (400R) gleitbar angeordnet sind,
Verhindern, dass sich der erste Ablenkungsdraht (131) und der zweite Ablenkungsdraht (142) innerhalb des Griffgehäuses kreuzen, und
Festhalten jedes von dem ersten Gleitelement (400L) und dem zweiten Gleitelement (400R) während der Translation innerhalb des Gleitfachs (700), um eine Bewegung quer zu einer Längsrichtung des Gleitfachs zu verhindern; und
eine Betätigungsbaugruppe (220), die antriebsmäßig mit jedem von dem ersten Gleitelement (400L) und dem zweiten Gleitelement (400R) gekoppelt ist, wobei die Betätigungsbaugruppe betätigt werden kann, um das erste Gleitelement (400L) distal und das zweite Gleitelement (400R) proximal innerhalb des Gleitfachs (700) gleichzeitig anzutreiben, und wobei die Betätigungsbaugruppe (220) betätigt werden kann, um das erste Gleitelement (400L) proximal und das zweite Gleitelement (400R) distal innerhalb des Gleitfachs gleichzeitig anzutreiben.

2. Lenkbare Einführvorrichtung nach Anspruch 1, wobei:
das Gleitchassis (300) eine Gleitelementöffnung (305) enthält, die sich über die Länge des Gleitchassis erstreckt; und
die Gleitelementöffnung (305) dazu konfiguriert ist, um die Installation mindestens eines von dem ersten Gleitelement (400L) und dem zweiten Gleitelement (400R) in das Gleitchassis zu ermöglichen.

3. Lenkbare Einführvorrichtung nach Anspruch 2, wobei:
das Gleitchassis (300) Tragkanten (305e) umfasst, die dazu konfiguriert sind, den Gleitblockteiler (500) zu tragen; und
die Gleitelementöffnung (305) mindestens teilweise durch die Tragkanten (305e) definiert ist.

4. Lenkbare Einführvorrichtung nach Anspruch 3, wobei das Gleitchassis (300) Schultern (311) zum Positionieren des Gleitblockteilers (500) enthält und sich die Schultern in einer Ebene senkrecht zu den Tragkanten (305e) an einem dem distalen Ende des Gleitchassis nahen Ende erstrecken.

5. Lenkbare Einführvorrichtung nach einem der Ansprüche 1 bis 4, wobei das Gleitchassis (300) im Wesentlichen zylindrisch geformt ist.

6. Lenkbare Einführvorrichtung nach einem der Ansprüche 1 bis 5, wobei der Gleitblockteiler (500, 1700) eine Finne (510, 1710) umfasst, die sich zu den Gleitelementen (400L, 400R) hin erstreckt, um das Rotieren der Gleitelemente zu verhindern und um zu verhindern, dass sich der erste Ablenkungsdraht (131) mit dem zweiten Ablenkungsdraht (142) innerhalb des Griffgehäuses kreuzt, optional
wobei die Finne (510, 1710) ein radial vorstehender Abschnitt ist, der sich von einem proximalen Ende des Gleitblockteilers zu einem distalen Ende des Gleitblockteilers erstreckt und zwischen dem ersten Gleitelement (400L) und dem zweiten Gleitelement (400R) angeordnet ist.

7. Lenkbare Einführvorrichtung nach einem der Ansprüche 1 bis 6, wobei:
die innere Oberfläche (238) des Griffgehäuses eine gekrümmte Form mit einer oder mehreren Rippen (233) aufweist, die voneinander beabstandet sind und sich entlang der gekrümmten Form erstrecken; und
die äußere Oberfläche (541) des Gleitblockteilers eine entsprechende gekrümmte Form mit Schlitzen (551, 552) aufweist, um die eine oder die mehreren Rippen (233) der inneren Oberfläche des Griffgehäuses aufzunehmen und eine relative Bewegung des Gleitblockteilers (500) gegenüber dem Griff (200) zu verhindern.

8. Lenkbare Einführvorrichtung nach einem der Ansprüche 1 bis 7, wobei:
der Gleitblockteiler (500) ein Paar von Schienen (521, 522) umfasst; und
jede des Paares von Schienen (521, 522) sich entlang einer Länge des Gleitblockteilers erstreckt und dazu konfiguriert ist, auf dem Gleitchassis aufzuliegen.

9. Lenkbare Einführvorrichtung nach Anspruch 8, wobei:
der Gleitblockteiler (500) Seitenwände (531, 533) umfasst, die sich entlang der Länge des Gleitblockteilers erstrecken; und
jede des Paares von Schienen (521, 522) an einer jeweiligen der Seitenwände (531, 533) ausgebildet ist.

10. Lenkbare Einführvorrichtung nach Anspruch 9, wobei die Seitenwände (531, 533) des Gleitblockteilers einen Heckabschnitt (536, 538) umfassen, der dazu konfiguriert ist, an einer Schulter (311) des Gleitchassis anzuliegen, um den Zusammenbau zu ermöglichen und eine relative Bewegung zwischen dem Gleitblockteiler und dem Gleitchassis zu verhindern, oder
wobei:
das Gleitchassis (300) flache Seitenabschnitte (313) umfasst, die sich mindestens teilweise entlang einer Länge des Gleitchassis erstrecken; und
innere Oberflächen der Seitenwände des Gleitblockteilers die flachen Seitenabschnitte kontaktieren.

11. Lenkbare Einführvorrichtung nach einem der Ansprüche 1 bis 10, wobei
das erste Gleitelement (400L) ein externes Rechtsgewinde (412) aufweist;
das zweite Gleitelement (400R) ein externes Linksgewinde (422) aufweist;
die Betätigungsbaugruppe einen drehbaren Knopf (221) umfasst, der dazu konfiguriert ist, ein Innengewinde (452) aufzuweisen, das dazu konfiguriert ist, mit dem externen Rechtsgewinde und dem externen Linksgewinde in Eingriff zu kommen.

12. Lenkbare Einführvorrichtung nach einem der Ansprüche 1 bis 11, wobei:
das erste Gleitelement (400L) Seitenoberflächen (416) am distalen Ende des ersten Gleitelements umfasst, die dazu konfiguriert sind, entlang Seitenoberflächen (512) des ersten Gleitelements des Gleitblockteilers zu gleiten, um das Rotieren des ersten Gleitelements innerhalb des Gleitfachs zu verhindern; und
das zweite Gleitelement (400R) Seitenoberflächen (426) am distalen Ende des zweiten Gleitelements umfasst, die dazu konfiguriert sind, entlang Seitenoberflächen (512) des zweiten Gleitelements des Gleitblockteilers zu gleiten, um das Rotieren des zweiten Gleitelements innerhalb des Gleitfachs zu verhindern.

13. Lenkbare Einführvorrichtung nach einem der Ansprüche 1 bis 12, wobei:
das erste Gleitelement (400L) einen ersten Ablenkungsdrahtkanal (401) umfasst, durch den sich der erste Ablenkungsdraht erstreckt; und
das zweite Gleitelement (400R) einen zweiten Ablenkungsdrahtkanal (411) umfasst, durch den sich der zweite Ablenkungsdraht erstreckt.

14. Lenkbare Einführvorrichtung nach Anspruch 13, die ferner Folgendes umfasst:
eine erste Stiftbaugruppe (1500A) mit einem ersten Stift (1503), der an einem proximalen Ende des ersten Ablenkungsdrahtkanals angeordnet ist, wobei der erste Ablenkungsdraht mit dem ersten Stift gekoppelt ist; und
eine zweite Stiftbaugruppe (1500B) mit einem zweiten Stift (1523), der an einem proximalen Ende des zweiten Ablenkungsdrahtkanals angeordnet ist, wobei der zweite Ablenkungsdraht mit dem zweiten Stift gekoppelt ist.

15. Lenkbare Einführvorrichtung nach Anspruch 14, wobei der Gleitblockteiler eine Finne (1710) umfasst, die sich radial zu den Gleitelementen (400L, 400R) hin erstreckt, wobei die Finne eine Höhe aufweist, die sich radial bis zum und über den ersten Stift und den zweiten Stift erstreckt, optional, wobei die Höhe der Finne (1710) entlang einer Längsachse des Gleitblockteilers konstant ist.

16. Lenkbare Einführvorrichtung nach einem der Ansprüche 13 bis 15, wobei das erste Gleitelement (400L) und das zweite Gleitelement (400R) durch einen Spalt (G1) voneinander getrennt sind, in den sich ein Abschnitt des Gleitblockteilers erstreckt, optional,
wobei:
der Gleitblockteiler (500) an einem proximalen Ende des Gleitblockteilers einen verlängerten Abschnitt (511) aufweist;
der verlängerte Abschnitt (511) in dem Spalt (G1) aufgenommen wird und verhindert, dass sich der erste Ablenkungsdraht (132) während des Betriebs der lenkbaren Einführvorrichtung mit dem zweiten Ablenkungsdraht (142) kreuzt.

## Revendications

1. Introducteur orientable (100) comprenant :
un corps allongé (110) comprenant une extrémité proximale (104), une section orientable (103), une première lumière de déflexion (131) s'étendant entre l'extrémité proximale (104) et la section orientable (103), et une seconde lumière de déflexion (132) s'étendant entre l'extrémité proximale (104) et la section orientable (103) ;
un premier fil de déflexion (141) s'étendant à l'intérieur de la première lumière de déflexion (131) et pouvant être actionné pour provoquer la déflexion de la section orientable du corps allongé dans une première direction ;
un second fil de déflexion (142) s'étendant à l'intérieur de la seconde lumière de déflexion (132) et pouvant être actionné pour provoquer la déflexion de la section orientable du corps allongé dans une seconde direction ;
un ensemble poignée (200) accouplé à l'extrémité proximale (104) du corps allongé, l'ensemble poignée comprenant :
un logement de poignée (230) comprenant une surface intérieure (238) ;
un châssis coulissant (300) disposé à l'intérieur du logement de poignée (230) ;
un premier élément coulissant (400L) accouplé par entraînement au premier fil de déflexion (131) ;
un second élément coulissant (400R) accouplé par entraînement au second fil de déflexion (142) ;
un diviseur de bloc coulissant (500) disposé à l'intérieur du logement de poignée et comprenant une surface externe (541) faisant interface avec la surface intérieure (238) du logement de poignée, dans lequel le diviseur de bloc coulissant (500) et le châssis coulissant (300) se combinent pour :
former un compartiment coulissant (700) dans lequel chaque élément parmi le premier élément coulissant (400L) et le second élément coulissant (400R) est disposé de manière coulissante,
empêcher le premier fil de déflexion (131) et le second fil de déflexion (142) de se croiser l'un l'autre à l'intérieur du logement de poignée, et
contraindre chaque élément parmi le premier élément coulissant (400L) et le second élément coulissant (400R), pendant la translation à l'intérieur du compartiment coulissant (700), pour empêcher un déplacement transversal par rapport à une direction longitudinale du compartiment coulissant ; et
un ensemble d'actionnement (220) accouplé par entraînement à chaque élément parmi le premier élément coulissant (400L) et le second élément coulissant (400R), dans lequel l'ensemble d'actionnement est utilisable pour simultanément entraîner le premier élément coulissant (400L) de manière distale et le second élément coulissant (400R) de manière proximale à l'intérieur du compartiment coulissant (700), et dans lequel l'ensemble d'actionnement (220) est utilisable pour simultanément entraîner le premier élément coulissant (400L) de manière proximale et le second élément coulissant (400R) de manière distale à l'intérieur du compartiment coulissant.

2. Introducteur orientable selon la revendication 1, dans lequel :
le châssis coulissant (300) comporte une ouverture pour élément coulissant (305) s'étendant sur une longueur du châssis coulissant ; et
l'ouverture pour élément coulissant (305) est conçue pour permettre l'installation d'au moins un élément parmi le premier élément coulissant (400L) et le second élément coulissant (400R) dans le châssis coulissant.

3. Introducteur orientable selon la revendication 2, dans lequel :
le châssis coulissant (300) comprend des bords de support (305e) conçus pour supporter le diviseur de bloc coulissant (500) ; et
l'ouverture pour élément coulissant (305) est au moins partiellement définie par les bords de support (305e).

4. Introducteur orientable selon la revendication 3, dans lequel le châssis coulissant (300) comporte des épaulements (311) pour le positionnement du diviseur de bloc coulissant (500), et les épaulements s'étendent dans un plan perpendiculaire aux bords de support (305e) à une extrémité proche de l'extrémité distale du châssis coulissant.

5. Introducteur orientable selon l'une quelconque des revendications 1 à 4, dans lequel le châssis coulissant (300) est de forme sensiblement cylindrique.

6. Introducteur orientable selon l'une quelconque des revendications 1 à 5, dans lequel le diviseur de bloc coulissant (500, 1700) comprend une ailette (510, 1710) s'étendant vers les éléments coulissants (400L, 400R) pour empêcher les éléments coulissants de tourner et pour empêcher le premier fil de déflexion (131) de croiser le second fil de déflexion (142) à l'intérieur du logement de poignée, éventuellement,
dans lequel l'ailette (510, 1710) est une partie faisant saillie radialement qui s'étend d'une extrémité proximale du diviseur de bloc coulissant à une extrémité distale du diviseur de bloc coulissant et est disposée entre le premier élément coulissant (400L) et le second élément coulissant (400R).

7. Introducteur orientable selon l'une quelconque des revendications 1 à 6, dans lequel :
la surface intérieure (238) du logement de poignée a une forme incurvée dotée d'une ou plusieurs nervures (233) espacées les unes des autres et s'étendant le long de la forme incurvée ; et
la surface externe (541) du diviseur de bloc coulissant a une forme incurvée correspondante dotée de fentes (551, 552) pour recevoir la ou les nervures (233) de la surface intérieure du logement de poignée et pour empêcher le déplacement relatif du diviseur de bloc coulissant (500) par rapport à la poignée (200).

8. Introducteur orientable selon l'une quelconque des revendications 1 à 7, dans lequel :
le diviseur de bloc coulissant (500) comprend une paire de rails (521, 522) ; et
chaque rail parmi la paire de rails (521, 522) s'étend sur une longueur du diviseur de bloc coulissant et est conçu pour reposer sur le châssis coulissant.

9. Introducteur orientable selon la revendication 8, dans lequel :
le diviseur de bloc coulissant (500) comprend des parois latérales (531, 533) s'étendant sur une longueur du diviseur de bloc coulissant ; et
chaque rail parmi la paire de rails (521, 522) est formé sur une paroi respective parmi les parois latérales (531, 533).

10. Introducteur orientable selon la revendication 9, dans lequel les parois latérales (531, 533) du diviseur de bloc coulissant comprennent une partie arrière (536, 538) conçue pour buter contre un épaulement (311) du châssis coulissant pour faciliter l'assemblage et pour empêcher le déplacement relatif entre le diviseur de bloc coulissant et le châssis coulissant, ou,
dans lequel :
le châssis coulissant (300) comprend des parties latérales plates (313) s'étendant au moins partiellement sur une longueur du châssis coulissant ; et
des surfaces intérieures des parois latérales du diviseur de bloc coulissant sont en contact avec les parties latérales plates.

11. Introducteur orientable selon l'une quelconque des revendications 1 à 10, dans lequel
le premier élément coulissant (400L) a un filet externe à droite (412) ;
le second élément coulissant (400R) a un filet externe à gauche (422) ;
l'ensemble d'actionnement comprend un bouton rotatif (221) ayant des filets internes (452) conçus pour venir en prise avec le filet externe à droite et le filet externe à gauche.

12. Introducteur orientable selon l'une quelconque des revendications 1 à 11, dans lequel :
le premier élément coulissant (400L) comprend des surfaces latérales d'extrémité distale de premier élément coulissant (416) conçues pour coulisser le long de surfaces latérales pour premier élément coulissant (512) du diviseur de bloc coulissant pour empêcher la rotation du premier élément coulissant à l'intérieur du compartiment coulissant ; et
le second élément coulissant (400R) comprend des surfaces latérales d'extrémité distale de second élément coulissant (426) conçues pour coulisser le long de surfaces latérales pour second élément coulissant (512) du diviseur de bloc coulissant pour empêcher la rotation du second élément coulissant à l'intérieur du compartiment coulissant.

13. Introducteur orientable selon l'une quelconque des revendications 1 à 12, dans lequel :
le premier élément coulissant (400L) comprend un canal pour premier fil de déflexion (401) à travers lequel le premier fil de déflexion s'étend ; et
le second élément coulissant (400R) comprend un canal pour second fil de déflexion (411) à travers lequel le second fil de déflexion s'étend.

14. Introducteur orientable selon la revendication 13, comprenant en outre :
un premier ensemble goupille (1500A) ayant une première goupille (1503) disposée à une extrémité proximale du canal pour premier fil de déflexion, dans lequel le premier fil de déflexion est accouplé à la première goupille ; et
un second ensemble goupille (1500B) ayant une seconde goupille (1523) disposée à une extrémité proximale du canal pour second fil de déflexion, dans lequel le second fil de déflexion est accouplé à la seconde goupille.

15. Introducteur orientable selon la revendication 14, dans lequel le diviseur de bloc coulissant comprend une ailette (1710) s'étendant radialement vers les éléments coulissants (400L, 400R), dans lequel l'ailette a une hauteur s'étendant radialement vers le bas jusqu'à la première goupille et la seconde goupille et au-dessus de celles-ci, éventuellement, dans lequel la hauteur de l'ailette (1710) est constante le long d'un axe longitudinal du diviseur de bloc coulissant.

16. Introducteur orientable selon l'une quelconque des revendications 13 à 15, dans lequel le premier élément coulissant (400L) et le second élément coulissant (400R) sont séparés par un espace (G1) dans lequel une partie du diviseur de bloc coulissant s'étend, éventuellement,
dans lequel :
le diviseur de bloc coulissant (500) a une partie étendue (511) à une extrémité proximale du diviseur de bloc coulissant ;
la partie étendue (511) est reçue dans l'espace (G1) et empêche le premier fil de déflexion (132) de croiser le second fil de déflexion (142) pendant le fonctionnement de l'introducteur orientable.
